(19) Europäisches Patentamt
European Patent Office
Office européen des brevets

(11) Veröffentlichungsnummer: **0 011 693**
**A1**

# (12) EUROPÄISCHE PATENTANMELDUNG

(21) Anmeldenummer: 79103734.4

(22) Anmeldetag: 01.10.79

(51) Int. Cl.³: C 07 D 233/36
C 07 D 233/70, C 07 D 233/72
C 07 D 239/22, C 07 D 249/12
C 07 D 263/20, C 07 D 263/44
C 07 D 275/04, A 01 N 43/50
A 01 N 43/54, A 01 N 43/64

(30) Priorität: 03.10.78 CH 10251/78

(43) Veröffentlichungstag der Anmeldung: 11.06.80 Patentblatt 80/12

(84) Benannte Vertragsstaaten: AT BE CH DE FR GB IT NL

(71) Anmelder: CIBA-GEIGY AG
Patentabteilung Postfach
CH-4002 Basel(CH)

(72) Erfinder: Tobler, Hans, Dr.
Baselmattweg 157
CH-4123 Allschwil(CH)

(72) Erfinder: Schurter, Rolf, Dr.
Holzmattstrasse 45
CH-4102 Binningen(CH)

(72) Erfinder: Töpfl, Werner, Dr.
Dorneckstrasse 68
CH-4143 Dornach(CH)

(72) Erfinder: Föry, Werner, Dr.
Benkenstrasse 65
CH-4054 Basel(CH)

(74) Vertreter: Zumstein sen., Fritz, Dr. et al,
Bräuhausstrasse 4
D-8000 München 2(DE)

## (54) Neue N-(trifluormethansulfonylaminophenyl)-substituierte N-Heterocyclen, ihre Herstellung, sie enthaltende Mittel und ihre Verwendung zur Beeinflussung des Pflanzenwachstums.

(57) Vorleigende Erfindung betrifft neue N-phenylsubstituierte N-Heterocyclen, ein Verfahren zu deren Herstellung sowie herbizide und pflanzenwachstumsregulierende Mittel, welche diese neuen Wirkstoffe als aktive komponente enthalten. Ferner betrifft die Erfindung die Verwendung dieser Wirkstoffe und Mittel zur Unkrautbekämpfung und pflanzenwachstumsregulierung.

Die neuen Wirkstoffe haben die Formel

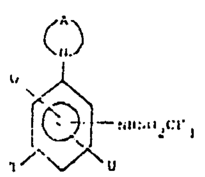

woring A drei bis sechs Glieder eines heterocyclischen Ringes darstellt, wobei mindestens eines und höchstens drei dieser Glieder gegebenenfalls substituierte Heteroatome der Gruppe 0, S, N und P sind und die restlichen gegebenenfalls substituierte Kohlenstoff-Kettenglieder darstellen, an die ein weiterer Ring ankondensiert sein kann, mit der Maßgabe, daß höchstens zwei vorhandene 0- bzw. S-Heteroatome benachbart stehen. Q, T und U bedeuten unabhängig voneinander Wasserstoff, Halogen, $C_{1-4}$-Alkyl, Halogenalkyl, Cyan, Nitro-, -$CSNH_2$, gegebenenfalls substituiertes Benzyl oder eine Gruppe -$S(0)_n$-R, -OR, -COOR, -$SO_2$-$N(R)_2$ oder -$N(R)_2$, wobei n eine Zahl von Null bis 2 ist und die Reste R jeweils gegebenenfalls substituierte Kohlenwasserstoffreste und zum Teil auch Wasserstoff bedeuten. Hergestellt werden die Verbindungen aus Zwischenprodukten, die anstelle der -$NHSO_2$ $CF_3$ Gruppe eine Aminogruppe tragen, z.B. 3-(4', 6'-Dimethyl-3'-aminophenyl)-imidazolidin-2-on, durch Behandlung mit einem Trifluormethansulfonylierungsmittel wie $(CF_3SO_2)_20$.

EP 0 011 693 A1

0011693



5-12060

BEZEICHNUNG GEÄNDERT, siehe Titelseite

CIBA-GEIGY AG, CH-4002 Basel / Schweiz

Neue N-phenylsubstituierte N-Heterocyclen, ihre Herstellung und Verwendung in Mitteln zur Beeinflussung des Pflanzenwachstums

Die vorliegende Erfindung betrifft neue N-phenylsubstituierte 4-bis 7-Ring N-Heterocyclen, Verfahren zu ihrer Herstellung, ferner Mittel zur Beeinflussung des Pflanzenwachstums, insbesondere herbizide und pflanzenwachstumshemmende Mittel, welche diese neuen Wirkstoffe als aktive Komponente enthalten, sowie Verfahren zur Beeinflussung des Pflanzenwachstums, insbesondere zur pre- und post-emergenten Unkrautbekämpfung und zur Hemmung des Pflanzenwachstums, unter Verwendung der neuen Wirkstoffe und der sie enthaltender Mittel.

Die neuen N-Heterocyclen vorliegender Anmeldung entsprechen der allgemeinen Formel I

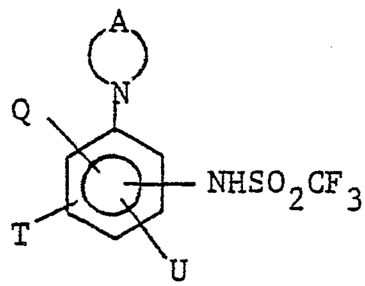

(I)

In dieser Formel bedeuten:

A drei bis sechs Glieder eines gesättigten oder ein- bis dreifach ungesättigten heterocyclischen Ringes, wobei mindestens eines und höchstens drei dieser Glieder gegebenenfalls substituierte Heteroatome aus den Gruppen O,S,N und P sind und die restlichen gegebenenfalls substituierte Kohlenstoff-Kettenglieder darstellen, wobei noch mindestens ein weiterer Ring ankondensiert vorhanden sein kann, mit der Massgabe, dass höchstens zwei vorhandene O- bezw. S-Atome benachbart stehen;

Q,T und U bedeuten unabhängig voneinander je Wasserstoff, Halogen, $C_1$-$C_4$ Alkyl oder Halogenalkyl, Cyano, Nitro, $-CS-NH_2$, gegebenenfalls substituiertes Benzyl oder eine Gruppe $-S(O)n-R_a$, $-OR_b$, $-COOR_c$, $-SO_2-N(R_d)_2$ oder $-N(R_e)_2$, wobei

n eine Zahl von Null bis 2,

$R_a$ $C_1$-$C_5$ Alkyl oder gegebenenfalls kernsubstituiertes Phenyl oder Phenylalkyl

$R_b$ $C_1$-$C_5$ Alkyl, Alkoxyalkyl, Alkoxyalkoxyalkyl, Alk(en)ylcarbonyl, oder gegebenenfalls durch niederesAlkyl, Halogen, CN, $CF_3$ oder $NO_2$ substituiertes Phenyl oder Benzyl,

$R_c$ Wasserstoff oder nieder Alkyl,

$R_d$ nieder ($C_1$-$C_4$) Alkyl, und

$R_e$ Wasserstoff oder $C_1$-$C_4$ Alkyl.

Der am obligatorischen Stickstoffatom des Heterocyclus befindliche, eben definierte Rest

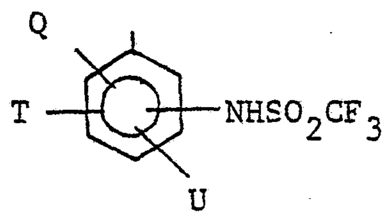

wird in der folgenden Beschreibung der Einfachkeit halber mit Ar bezeichnet. Die $-NHSO_2CF_3$ Gruppe befindet sich vorzugsweise in meta-Stellung, kann aber auch in para-Stellung stehen.

Die zusätzlichen Heteroatome des 4-bis 7-gliedrigen heterocyclischen Ringes

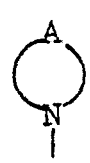

können, falls sie 3- bis 6-wertige Heteroatome sind (N, S, P), noch substituiert sein, also Gruppen wie

$>N-R_1$, $>S(O)_n$ oder $>\overset{L}{P}=Z$ darstellen, wobei $R_1$ ein später noch näher definierter Substituent, n eine Zahl von Null bis 2, Z Sauerstoff oder Schwefel und L eine Gruppe $-OR_4$ oder $-N{<}^{R_6}_{R_7}$ ist, wobei die Reste $R_4$, $R_6$ und $R_7$ später näher definiert werden.

Zwei benachbarte Ringglieder (C oder N) können mit einer $C_3$-$C_4$-Alkylen-, $C_3$-$C_4$-Alkenylen- oder $C_4$-Alkadienylkette einen zusätzlichen ankondensierten Ring bilden. Die verbleibenden Kohlenstoffglieder des heterocyclischen Ringes können noch eine oder mehrere der folgenden Gruppen tragen: Alkyl, Cycloalkyl, Aralkyl, Aryl, Alkoxy, Alkoxyalkoxy, Alkyl- und Alkenyl-carbonyloxy, Alkylthio, Carboxyl, Alkoxycarbonyl, Benzoyloxy, Benzylthio, Phenylthio, Halogen, Cyano, =O, =S, $=NR_4$ oder $-N{<}^{R_6}_{R_7}$ .

Die wichtigsten Untergruppen stellen Heterocyclen der allgemeinen Formel IIa oder IIb

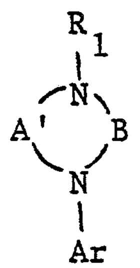 (IIa)

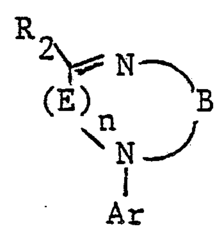 (IIb)

dar, worin die neuen und die schon früher verwendeten Symbole folgende Bedeutung haben:

Ar steht für den substituierten Phenylrest der Formel I

A' ist eine Gruppe $>C=O$, eine gegebenenfalls durch $R_2$ oder $R_3$ substituierte $C_1$-$C_3$ Alkylen- oder Alkenylen-Gruppe oder ein Rest -CO-D, worin D gegebenenfalls durch $R_2$ und/oder $R_3$ substituiertes $C_1$-$C_2$ Alkylen oder $C_2$-Alkenylen darstellt, $R_2$ und $R_3$ neben Wasserstoff einen gegebenenfalls durch $C_1$-$C_4$ Alkoxy oder durch CN substituierten $C_1$-$C_8$ Alkyl, $C_1$-$C_4$ Alkoxy-, einen Cyano- oder $-S(O)_n$-Alkylrest bedeuten, n eine Zahl von Null bis 2 ist, $R_2$ und $R_3$ zusammen auch einen $C_2$-$C_6$ Alkylen, $C_2$-$C_6$ Alkenylen oder $C_4$-Alkadienylenrest zwecks Bildung eines weiteren Ringes bedeuten können, der selbst wiederum durch $R_2$ bezw. $R_3$ substituiert sein kann,

B hat entweder die gleiche Bedeutung wie A' oder ist eine Gruppe -CO-CO- oder $>C=X$, worin X Schwefel oder $=NR_4$ bedeutet;

E ist gegebenenfalls durch $R_2$ und/oder $R_3$ substituiertes $C_2$-Alkenylen, die Gruppe $>C=O$ oder $>C<^{R_2}_{R_3}$ und n ist eine Zahl von Null bis 2,

$R_1$ ist $C_1$-$C_8$ Alkyl (auch $R_5$ genannt), Wasserstoff, $C_3$-$C_8$ Cycloalkyl, wobei Alkyl- und Cycloalkylreste gegebenenfalls substituiert sein können durch Halogen, $C_1$-$C_4$ Alkoxy, durch $-N<^{R_7}_{R_6}$, CN, $-COOR_8$, oder gegebenenfalls substituiertes Phenyl, $C_3$-$C_8$ Alkenyl oder Halogenalkenyl, $C_3$-$C_8$ Alkinyl, gegebenenfalls durch Alkyl, Alkoxy, Halogen, $CF_3$ oder CN substituiertes Phenyl, ferner eine der Gruppen $-CON<^{R_6}_{R_7}$, $-COOR_8$ oder $COR_1$, wobei $R_1$ wieder die obige Bedeutung hat,

$R_4$ ist $C_1$-$C_8$ Alkyl, Benzyl oder Phenyl,

$R_6$ und $R_7$ bedeuten unabhängig voneinander je Wasserstoff, gegebenenfalls durch $C_1$-$C_4$ Alkoxy substituiertes $C_1$-$C_8$ Alkyl, $C_3$-$C_8$ Alkenyl oder Alkinyl, $C_1$-$C_4$ Alkoxy, Phenyl oder Benzyl, wobei der Benzolkern dieser Reste durch Halogen, $C_1$-$C_4$ Alkyl, $C_1$-$C_4$ Alkoxy oder durch CN substituiert sein kann,

$R_6$ und $R_7$ können zusammen mit dem benachbarten Stickstoffatom auch einen 5-6-gliedrigen heterocyclischen Ring bilden, der noch ein weiteres Sauerstoffatom als Heteroatom enthalten kann,

$R_8$ ist Wasserstoff, $C_1$-$C_8$ Alkyl, $C_3$-$C_8$ Cycloalkyl, gegebenenfalls durch $C_1$-$C_4$ Alkoxy oder Halogen substituiertes Aralkyl (insbesondere Benzyl oder Phenyläthyl), ferner gegebenenfalls durch Halogen, Alkyl, Alkoxy, $CF_3$ oder CN substituiertes Phenyl.

Unter den Heterocyclen der allgemeinen Formel II sind folgende Typen besonders hervorzuheben:

<u>1-Aryl-uretidin-2,4-dione</u> der Formel

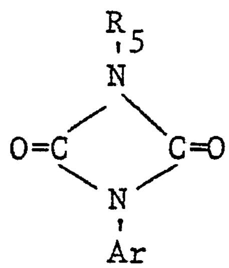

$R_5 = C_1\text{-}C_8$ Alkyl

(3)

<u>1-Aryl-imidazolidin-2-one</u> der Formel

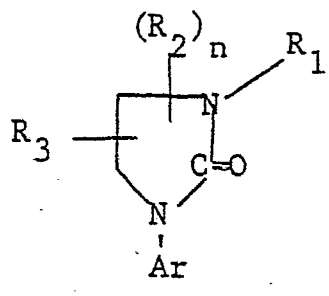

(4)

<u>3-Aryl-imidazolidin-2,4-dione</u> der Formel

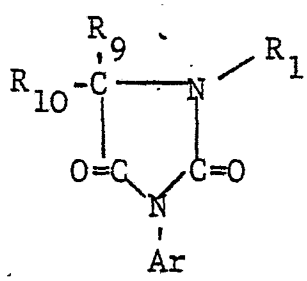

(5)

worin $R_9$ und $R_{10}$ je <u>Wasserstoff</u>, $C_1$-$C_8$ <u>Alkyl</u>, $C_3$-$C_8$ <u>Cycloalkyl</u>, gegebenenfalls durch Alkoxy oder Halogen substituiertes <u>Aralkyl</u> oder gegebenenfalls durch Alkyl, Halogen, $C_1$-$C_4$ Alkoxy, $CF_3$ oder CN substituiertes <u>Phenyl</u> bedeuten,

$R_9$ und $R_{10}$ zusammen aber auch einen 5- oder 6-gliedrigen gesättigten oder ungesättigten Ring bilden können, der noch durch $C_1$-$C_8$ Alkyl substituiert sein kann.

1-Aryl-imidazolidin-2,4-dione der Formel

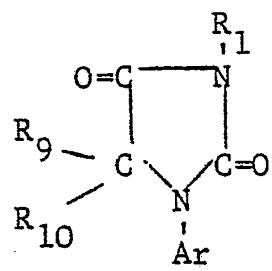

(6)

1-Aryl-imidazolidin-2,4,5-trione der Formel

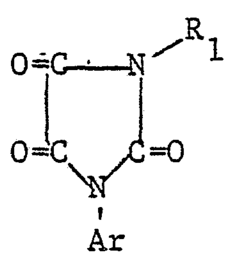

(7)

1-Aryl-imidazolin-2-one der Formel

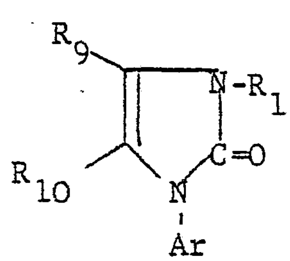

(8)

1-Aryl-imidazole der Formel

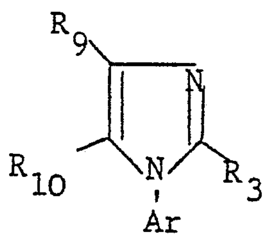

(9)

1-Aryl-imidazolin-4-one der Formel

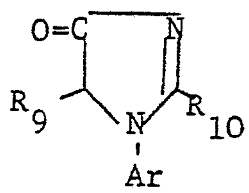

(10)

1-Aryl-1,4,5,6-tetrahydro-pyrimidin-2-one der Formel

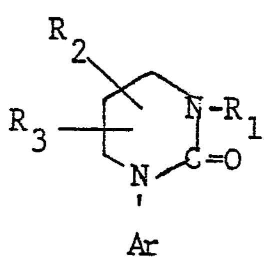

(11)

1-Aryl-hexahydro-pyrimidine der Formel

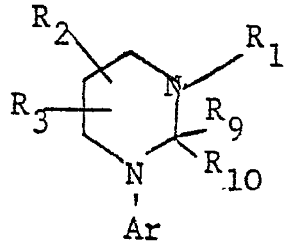

(12)

1-Aryl-pyrimidin-2-one der Formel

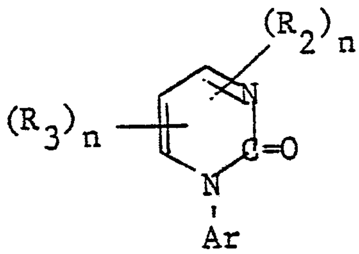

(13)

1-Aryl-1,4-dihydro-pyrimidin-4-one der Formel

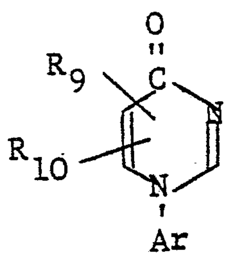

(14)

Aryl-uracile der Formeln

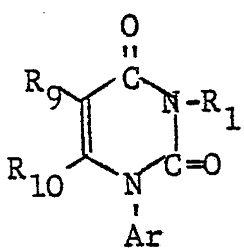

(15) und

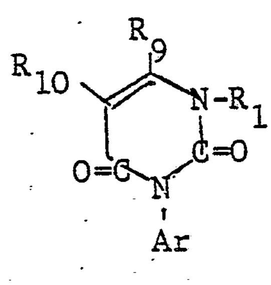

(16)

<u>1-Aryl-5,6-dihydrouracile</u> der Formel

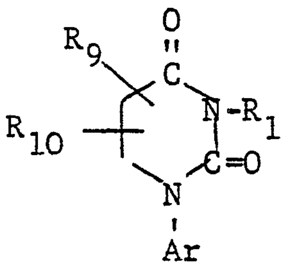

(17)

<u>1-Aryl-4,5-dihydro-uracile</u> der Formel

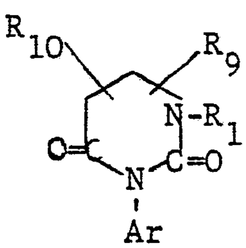

(18)

<u>1-Aryl-piperazin-2,6-dione</u> der Formel

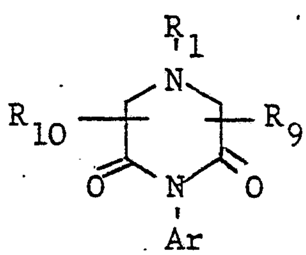

(19)

1-Aryl-piperazin-2,5-dione der Formel

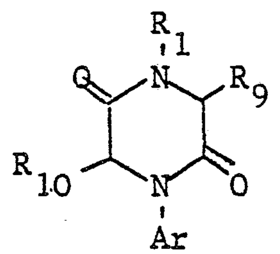

(20)

1-Aryl-piperazin-2,3-dione der Formel

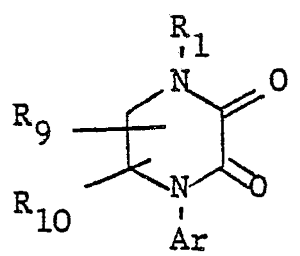

(21)

1-Aryl-dehydro-piperazin-2-one der Formel

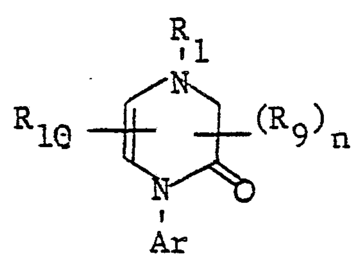

(22)

1-Aryl-piperazin-2,3,5-trione der Formel

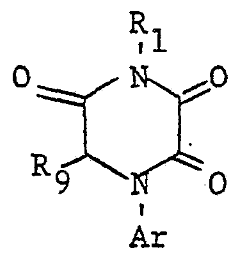

(23)

1-Aryl-piperazin-2,3,6-trione der Formel

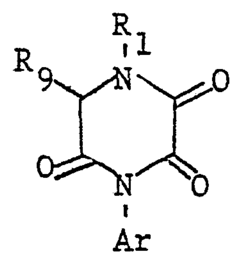

(24)

1-Aryl-piperazin-2,3,5,6-tetrone der Formel

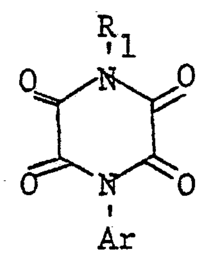

(25)

Verbindungen der allgemeinen Formel

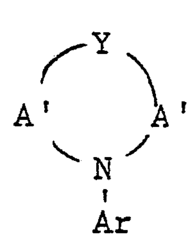

(26)

worin Y Sauerstoff, Schwefel, -SO- oder $-SO_2-$ darstellt,

3-Aryl-oxazolidin-2-one der Formel

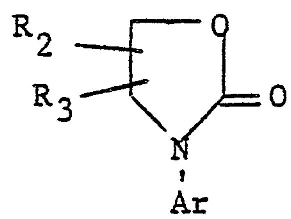

(27)

3-Aryl-oxazolidine der Formel

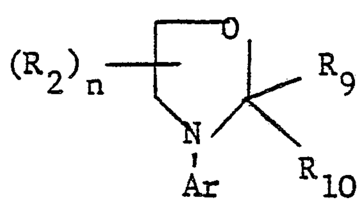

(28)

3-Aryl-oxazolidin-2,4-dione der Formel

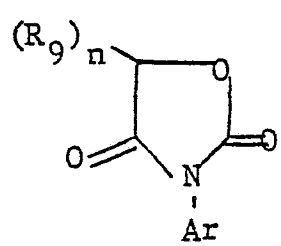

(29)

3-Aryl-thiazolidin-2-one der Formel

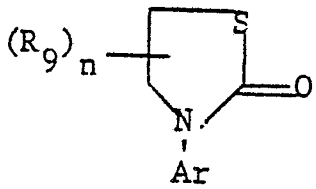

(30)

3-Aryl-thiazolidin-2,4-dione der Formel

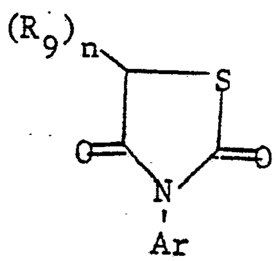

(31)

3-Aryl-thiazolin-2-one der Formel

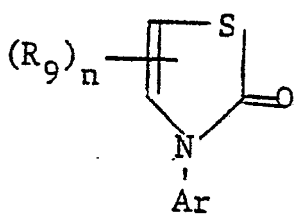

(32)

Verbindungen des Typs

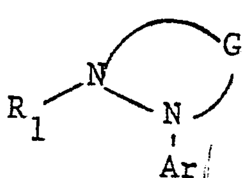

(33a)

oder

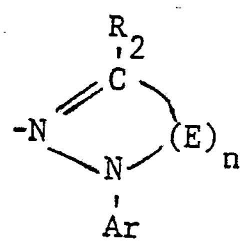

(33b)

worin G entweder die Bedeutung von A' hat oder die Gruppe $\geq C=X$ darstellt (X ist = S oder $=N-R_4$)

1-Aryl-6(1H)-pyridazinone der Formeln

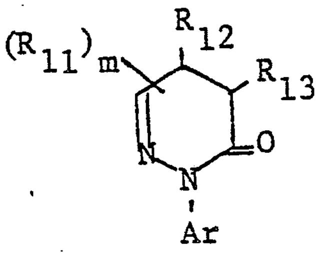

oder

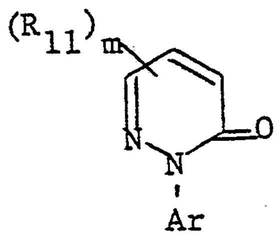

(34) (34a)

worin $R_{11}$ die Bedeutungen von $R_1$ hat oder eine Gruppe 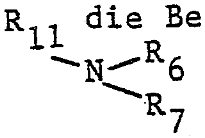 ist, m eine Zahl von 0 bis 3 und $R_{12}$ und $R_{13}$ dasselbe bedeuten wie $R_{11}$.

1-Aryl-pyridazin-3,6-dione der Formel

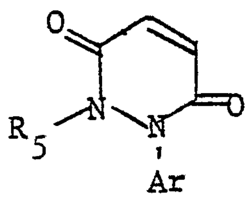

(35)

1-Aryl-4,5-dihydro-pyridazin-3,6-dione der Formel

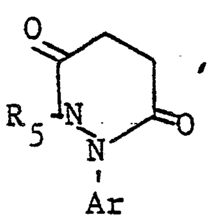

(36)

1-Aryl-pyridazin-4-one der Formel

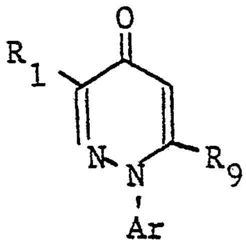

(37)

1-Aryl-pyridazin-4,6-dione der Formel

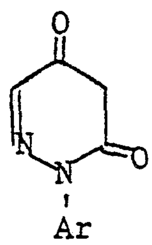

(38)

1-Aryl-pyrazole der Formel

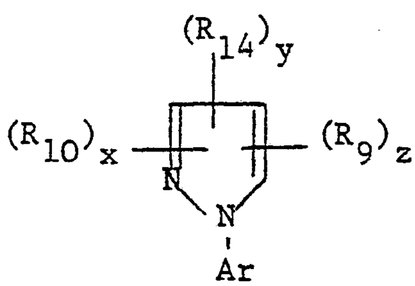

(39)

worin $R_{14}$ dasselbe wie $R_{11}$ oder Halogen, -O-Acyl und -NH-Acyl bedeutet und die Summe x+y+z höchstens 3 ist.

1-Aryl-pyrazolidine der Formel

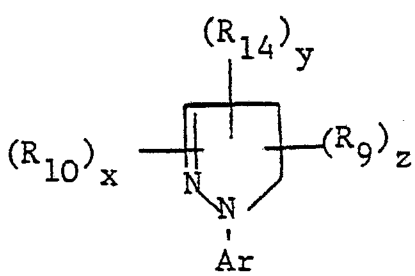

(40)

mit denselben Definitionen wie unter (39)

<u>1-Aryl-2-pyrazolin-5-one</u> der Formel

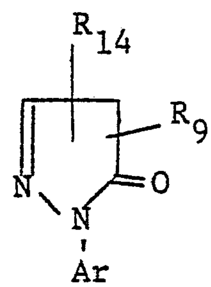

(41)

<u>1-Aryl-3-pyrazolin-5-one</u> der Formel

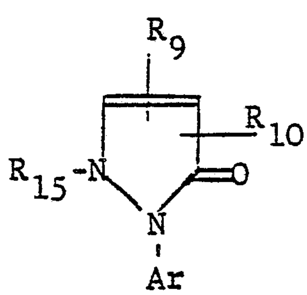

(42)

worin $R_{15}$ Wasserstoff, $C_1$-$C_6$ Alkyl oder $C_1$-$C_8$ Alkylcarbonyl bedeutet.

<u>1-Aryl-pyrazolidin-3-one</u> der Formel

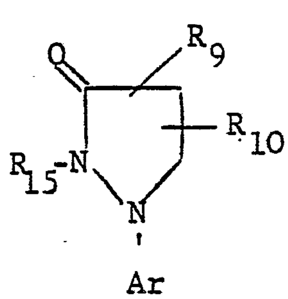

(43)

1- Aryl-pyrazolidin-5-one der Formel

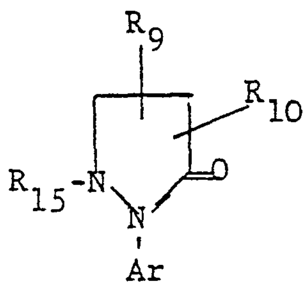

(44)

1-Aryl-pyrazolin-3-one der Formel

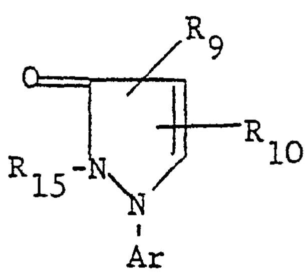

(45)

1-Aryl-pyrazolin-4-one der Formel

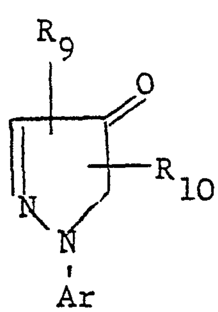

(46)

1-Aryl-pyrazolidin-3,5-dione der Formel

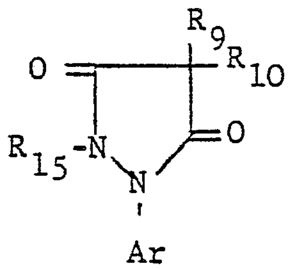

(47)

Sultame der Formeln

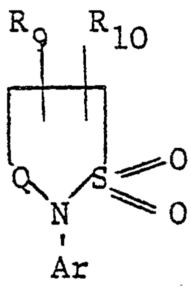

(48)

worin Q entweder $-CH_2-$ oder $\geq C{=}O$ bedeutet und

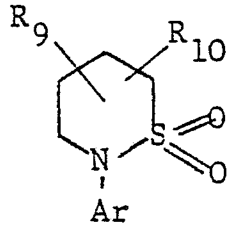

(49)

5- und 6-gliedrige Heterocyclen mit 3 Heteroatomen:

4-Aryl-urazole der Formel

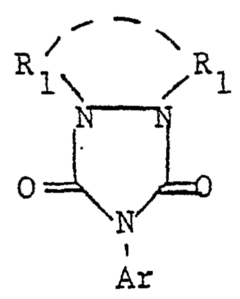

(50)

4-Aryl-1,2,4-triazolidin-3-one der Formel

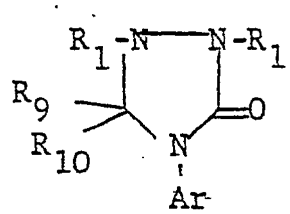

(51)

4-Aryl-1,2,4-triazole der Formel

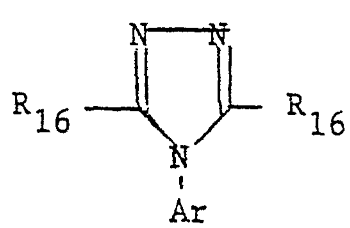

(52)

worin $R_{16}$ dieselbe Bedeutung wie $R_{15}$ hat oder Alkoxy bzw. Alkylthio bedeutet.

4-Aryl-1,2,4-triazolin-3-one der Formel

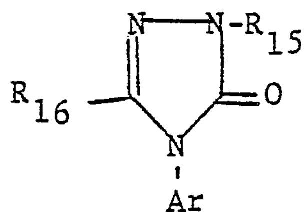

(53)

1-Aryl-1,2,4-triazole der Formel

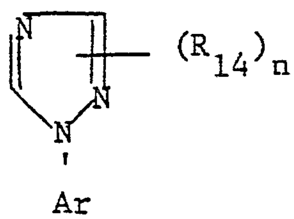

(54)

1-Aryl-1,2,3-triazole der Formel

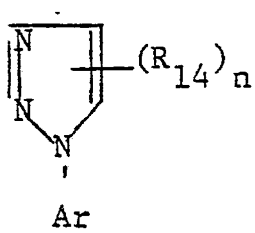

(55)

<u>3-Aryl-1,3,4-oxa-(und thia)-diazolin-2-one</u>
der Formel

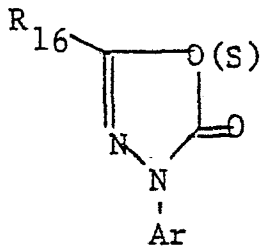

(56)

<u>2-Aryl-1,2,3-thiadiazolin-S-dioxide</u> der Formel

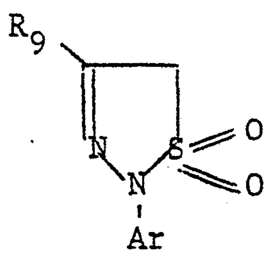

(57)

<u>2-Aryl-tetrahydro-1,2,4-triazin-3,5-dione</u>
der Formel

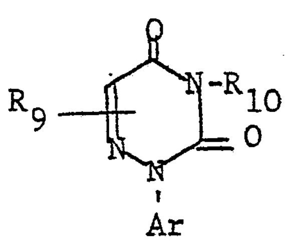

(58)

1-Aryl-tetrahydro-1,3,5-triazin-2,6-dione der Formel

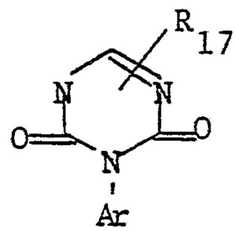

(59)

worin $R_{17}$ die gleiche Bedeutung wie $R_9$ hat oder eine Gruppe $-N{<}^{R_6}_{R_7}$ darstellt.

1-Aryl-hexahydro-1,3,5-triazin-2-one der Formel

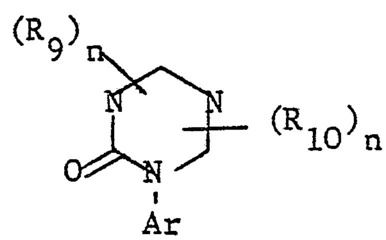

(60)

4-Aryl-dihydro-1,3,4-oxa-(und thia)-diazin-5-one der Formel

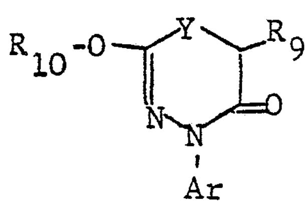

(61)

worin Y Sauerstoff oder $S(O)_n$ bedeutet, und n eine Zahl von Null bis 2 ist.

Der in den vorstehenden Formeln vorkommende Rest Ar, welcher dem substituierten Phenylrest

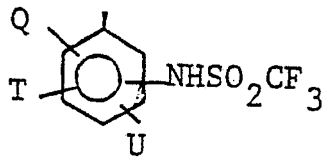

entspricht, ist vorzugsweise einer der folgenden 9 Reste:

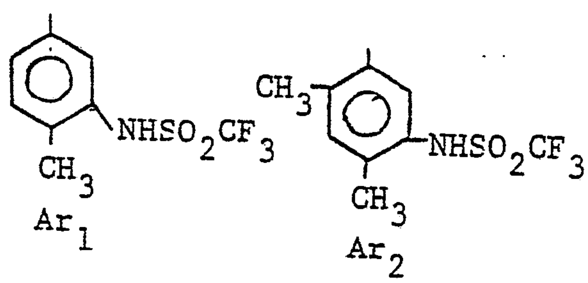

$Ar_1$ $Ar_2$

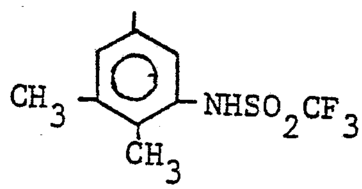

$Ar_3$

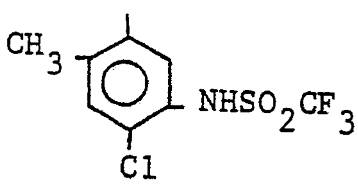

$Ar_4$

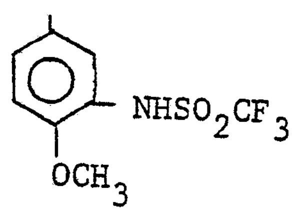

$Ar_5$

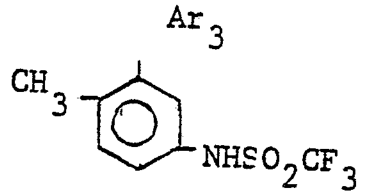

$Ar_6$

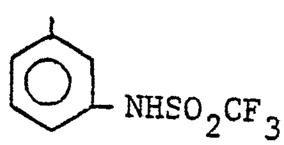

$Ar_7$

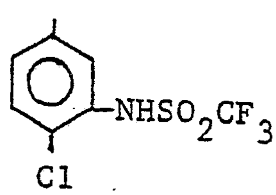

$Ar_8$

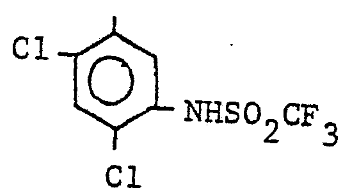

$Ar_9$

welche in der Beschreibung und den Tabellen mit $Ar_1$ bis $Ar_9$ bezeichnet werden.

Die $-NHSO_2CF_3$-Gruppe der neuen Wirkstoffe der vorstehenden allgemeinen Formeln I, IIa und IIb und der verschiedenen Untergruppen der Formeln (3)bis(61) ist befähigt, Salze zu bilden, in denen das Wasserstoffatom durch ein Kation ersetzt ist, vorzugsweise durch Natrium oder Kalium oder ein organisches Amin-Kation. Unter organischen Aminen hat sich Diäthanolamin besonders bewährt.

Als Metallkationen kommen auch die von Erdalkalimetallen, von Zink, Kupfer, Eisen etc. in Betracht. Ist das vorhandene Kation 2- oder 3-wertig, so ist es selbstverständlich mit der seiner Wertigkeit entsprechenden Anzahl Anionen des Basiskörpers I liiert. Auf den Grundkörper I entfällt dann $\frac{1}{m}$ Kation der Wertigkeit m.

Aus den USA-Patentschriften 3 238 222 und 3 958 974 sind bereits herbizid wirksame N-phenylsubstituierte Pyrrolidin-2-one, Piperidin-2-one und Azetidin-2-one bekannt geworden, die aber im Phenylring anders substituiert sind.

Andererseits sind in der USA-Patentschrift 3 920 444 und in der DOS 2 364 144 herbizid wirksame Perfluoralkansulfonanilid-Derivate beschrieben worden, welche aber keine heterocyclische Gruppierung aufweisen.

Es wurde überraschenderweise gefunden, dass die erfindungsgemässen neuen heterocyclischen Wirkstoffe der Formel I den aus obigen Literaturstellen bekannten Wirkstoffen klar überlegen sind, sowohl inbezug auf herbizide Wirkung als inbezug auf Eignung als Pflanzenwuchshemmer.

Das Verfahren zur Herstellung der neuen Heterocyclen der Formel I ist dadurch gekennzeichnet, dass man ein heterocyclisches Amino-anilinderivat der Formel III

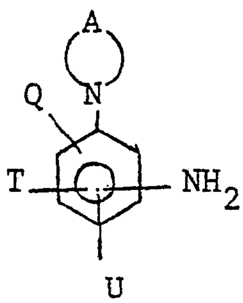

(III)

worin A, Q, T und U wie unter Formel I definiert sind, in an sich bekannter Weise mit einem Trifluormethansulfonylierungsmittel in Gegenwart eines Säureakzeptors behandelt und das so erhaltene Trifluormethansulfonamid gewünschtenfalls in das Salz einer Base oder eines Amins überführt.

Als Mittel zur Einführung der Trifluormethansulfonsäuregruppe kann das Anhydrid der Trifluormethansulfonsäure $(CF_3SO_2)_2O$ oder ein Halogenid dieser Säure der Formel $CF_3$-$SO_2$-Hal dienen, wo Hal vorzugsweise Fluor oder Chlor bedeutet.

Als Säureakzeptoren dienen die für solche Acylierungen üblichen Ammoniumbasen, Alkali- und Erdalkali-hydroxide, -Carbonate, -Hydrogencarbonte, sowie primäre, sekundäre und tertiäre Amine, wie z.B. Triäthylamin oder N,N-Dimethylanilin.

Die Ausgangsamine der Formel III sind zum Teil ebenfalls neue, in der Literatur noch nicht beschriebene Stoffe.

Alle heterocyclischen Ringtypen, die in den Formeln (3) bis (61) vorkommen, sind hingegen bekannt und ihre Herstellung nach bekannten Methoden ist in vielen Literaturstellen beschrieben.

Im Prinzip erfolgt die Herstellung der Zwischenprodukte der Formel III durch Anbau des bekannten Heterocyclus an ein Ausgangsanilin der Formel IV

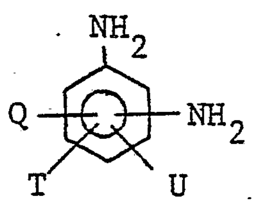 (oder $NO_2$) (IV)

Verwendet man als Ausgangsstoff der Formel IV ein Nitroanilin, was oft vorzuziehen ist, um die Bildung von Stellungsisomeren zu vermeiden, so muss nach dem Anbau des Heterocyclus an die 1-ständige Aminogruppe die Nitrogruppe durch Hydrierung oder Reduktion nach wohlbekannten Methoden in die Aminogruppe umgewandelt werden.

Falls in den Zwischenprodukten der Formel III der Heterocyclus durch Halogen substituiert ist, so kann dieses Halogen durch Umsetzung mit KCN oder einem Alkanolat durch die Cyanogruppe oder eine Alkoxygruppe ersetzt werden.

Alle Umsetzungen werden vorzugsweise in gegenüber den Reaktionsteilnehmern inerten Lösungs- und Verdünnungsmitteln durchgeführt.

Bevorzugt sind polare organische Lösungsmittel, wie Alkohole, Ketone, Dimethylformamid, Tetrahydrofuran, Dimethylsulfoxyd etc.

In den nachfolgenden Beispielen wird die Herstellung einiger erfindungsgemässer Wirkstoffe der allgemeinen Formel I und ihrer dazu benötigten Zwischenprodukte beschrieben. Weitere hergestellte Endstoffe sind, teilweise unter Angabe der Literatur zur Herstellung der Zwischenprodukte, in den anschliessenden Tabellen aufgeführt. Alle Temperaturangaben beziehen sich auf Celsius-Grade.

Beispiel 1

1-(4',6'-Dimethyl-3'-trifluormethansulfonamido-phenyl)-imidazolidin-2-on

[Typus Formel (4)]

a)

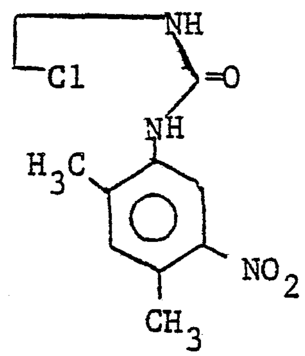

Zu einer Lösung von 166 g (1,0 Mol) 2,4-Dimethyl-5-nitroanilin in siedendem Acetonitril (1 Liter) tropft man 116,1 g 2-Chlor-äthylisocyanat (1,1 Mol) hinzu. Nach 15 Minuten kühlt man ab und filtriert das auskristallisierte Produkt. Man wäscht das Produkt mit kaltem Acetonitril. Nach dem Trocknen (70°) erhält man 200 g (73,7%) N-Chloräthyl-N'-(4,6-dimethyl-3-nitrophenyl)-harnstoff, Smp. 186–187°C.

b)

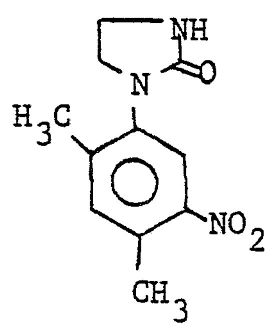

Obiges Produkt (200 g, 0,737 Mol) wird in 400 ml 15 prozentiger Natronlauge und 40 ml Tetrahydrofuran während 4 Std. am Rückfluss gekocht. Man kühlt auf 10°C und stellt die

Mischung sauer (pH1). Filtration und Umkristallisation liefert 98,9 g (57,1%) 3-(4',6'-Dimethyl-3'-nitrophenyl)-imidazolidin-2-on, Smp. 140°C.

$C_{11}H_{13}N_3O_3$ [235,24]

| | | | | | | |
|---|---|---|---|---|---|---|
| Ber. | C | 56,17 | H | 5,57 | N | 17,87 % |
| Gef. | C | 55,9 | H | 5,6 | N | 18,0 % |

c)

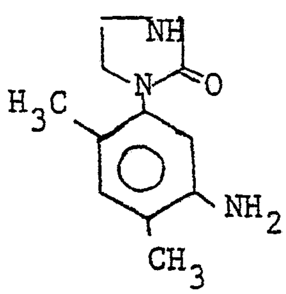

Obiges Produkt (50 g, 0,213 Mol) wird in 500 ml abs. Methanol mit 5 g RaNi bei 35°C hydriert. Die heiss filtrierte Lösung wird auf 200 ml eingeengt und abkühlen gelassen. Das Produkt kristallisiert aus. Man erhält 32,3 g (74,1%) 3(4',6'-Dimethyl-3'-aminophenyl)-imidazolidin-2-on, Smp. 222°C.

$C_{11}H_{15}N_3O$ [205,26]

| | | | | | | |
|---|---|---|---|---|---|---|
| Ber. | C | 64,37 | H | 7,37 | N | 20,47 % |
| Gef. | C | 64,4 | H | 7,5 | N | 20,3 % |

d)

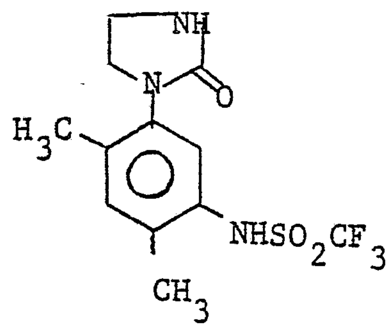

Obiges Produkt (15,0 g, 0,073 Mol) und 9,3 g N,N-Dimethylanilin (1,05 Aequ.) in 300 ml Chloroform abs. werden bei -5°C innert 35 Minuten mit 22,6 g $(CF_3SO_2)_2O$ (1,05 Aequ) topfenweise versetzt. Man lässt die Temperatur auf Raumtemperatur ansteigen und extrahiert die Suspension 3 mal mit je 150 ml 1N NaOH. Der basische Auszug wurde filtriert und sauer gestellt (pH 1) mit conc. HCl. Das so gefällte Produkt wurde abfiltriert, gewaschen ($H_2O$) und getrocknet (70°). Ausbeute 21,0 g (85,4%), Smp. 288°C. $C_{12}H_{14}F_3N_3O_3S$ [337,32]

| | | | | | | | | | | |
|---|---|---|---|---|---|---|---|---|---|---|
| Ber. | C | 42,73 | H | 4,18 | N | 12,46 | S | 9,51 | F | 16,90% |
| Gef. | C | 42,0 | H | 4,2 | N | 12,3 | S | 9,9 | F | 16,9 % |

Beispiel 2

1-(4',6'-Dimethyl-3'-trifluormethansulfonamido-phenyl)-3-methyl-imidazolidin-2-on

[ Typus Formel (4) ]

a)

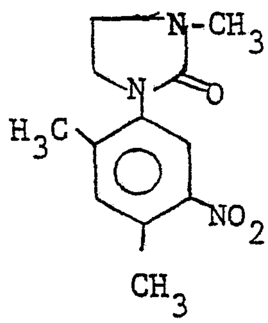

45 g (0,2 Mol) 3-(4',6'-Dimethyl-3'-nitrophenyl)-imidazolidin-2-on (Beispiel 1b) und 6,5 g (0,02 Mol) Tetrabutylammoniumbromid werden in 500 ml Benzol vorgelegt. Man fügt dann 40 g festes NaOH in 40 ml $H_2O$ zu. Nach 15 Min. Rühren fügt man tropfenweise 32 g Dimethylsulfat (0,24 Mol) hinzu. Die Temperatur steigt dabei auf 40°C. Nach 2 Std. Rühren versetzt man die Reaktionsmischung mit $H_2O$ und trennt die wässerige Phase ab. Die organische Phase wurde sukzessive mit 2N HCl, gesätt. $NaHCO_3$-Lösung, $H_2O$ und gesätt. NaCl-Lösung gewaschen, mit $MgSO_4$ getrocknet und eingedampft. Der ölige Rückstand wurde mit Aether verrieben und aus Essigester umkristallisiert. 38 g (80%) 1-(4',6'-Dimethyl-3'-nitrophenyl)-3-methyl-imidazolidin-2-on vom Smp. 107-109°C werden so erhalten.

$C_{12}H_{15}N_2O_3$ [249,27]

| | | | | | | |
|---|---|---|---|---|---|---|
| Ber. | C | 57,82 | H | 6,07 | N | 16,86 % |
| Gef. | C | 57,7 | H | 6,1 | N | 16,7 % |

b)

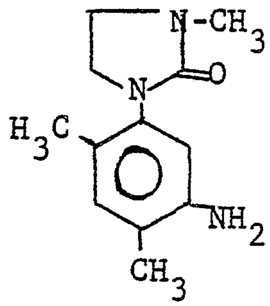

36,1 g (0,145 Mol) obigen Produktes werden in 360 ml Methanol mit 4 g Raney-Nickel bei 35°C hydriert. Nach Filtration des Gemisches und Einengen der Lösung kristallisieren 26,2 g (82%) 1-(4',6'-Dimethyl-3'-amino-phenyl)-3-methyl-imidazolidin-2-on, Smp. 119-120°C.

$C_{12} H_{17} N_3 O$ [ 219,29]

| | | | | | | |
|---|---|---|---|---|---|---|
| Ber. | C | 65,73 | H | 7,82 | N | 19,16 % |
| Gef. | C | 65,7 | H | 7,9 | N | 19,4 % |

c)

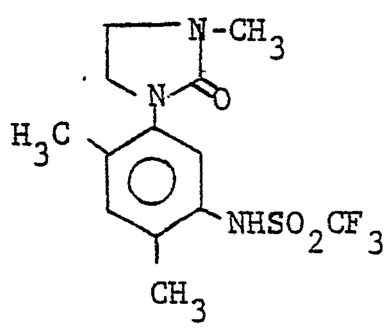

10 g (0,047 g Mol) obigen Zwischenproduktes und 6,2 g N,N-Dimethylanilin (1,05 Aequ.) in 100 ml abs. Chloroform werden bei -5° tropfenweise mit 14,5 g $(CF_3SO_2)_2O$ (1,07 Aequ.) mit 9 ml Chloroform versetzt. Nach Beendigung der Zugabe wurde ohne Kühlung während 2 Std. weitergerührt. Man extrahiert die Reaktionslösung

2 mal mit je 500 ml 1N NaOH. Die vereinigten basischen Auszüge wurden mit Chloroform gewaschen, filtriert und mit Salzsäure sauer gestellt (pH 1). Das auf diese Weise ausgefällte Produkt wurde abfiltriert, mit Wasser gewaschen und bei 70° im Exsikkator getrocknet: man erhält 15,5 g (96,5%) 1-(4',6'-Dimethyl-3'-trifluormethansulfon-amido-phenyl)-3-methyl-imidazolidin-2-on vom Smp. 226-230°.

$C_{13}H_{16}F_3N_3O_3S$ [351,34]

| | | | | | | | | | | |
|---|---|---|---|---|---|---|---|---|---|---|
| Ber. | C | 44,44 | H | 4,59 | N | 11,96 | S | 9,13 | F | 16,22% |
| Gef. | C | 44,3 | H | 4,6 | N | 11,9 | S | 9,2 | F | 16,1% |

Beispiel 3

3-(4',6'-Dimethyl-3'-trifluormethansulfonamido-phenyl)-imidazolidin-2,4-dion

[Typus Formel (5)]

a)

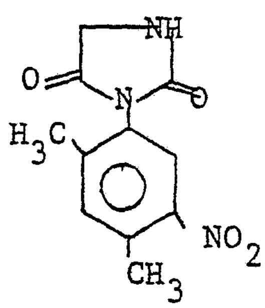

11,2 g (0,15 Mol) Glycin in 100 ml $H_2O$ wurden mit 6 g fester Natronlauge neutralisiert. Unter Rühren fügt man tropfenweise bei Raumtemperatur 28,8 g (0,15 Mol) 4,6-Dimethyl-3-nitro-phenylisocyanat zu. Es resultierte eine hellgelbe Lösung. Nach 4 Std. Reaktionszeit fiel ein Festkörper aus, welcher abfiltriert, mit Essigester gewaschen, und hernach

in conc. Salzsäure am Rückfluss gekocht wurde. Nach 3 Std. goss man auf Eiswasser, filtrierte das Produkt ab und kristallisierte es am Aethanol aus. Man erhielt 17,3 g (46%) 3-(4',6'-Dimethyl-3'-nitrophenyl)-imidazolidin-2,4-dion, Smp. 174-175°C.

$C_{11} H_{11} N_3 O_4$ [249,33]

| | | | | | | |
|---|---|---|---|---|---|---|
| Ber. C | 53,01 | H | 4,45 | N | 16,86 % |
| Gef. C | 53,2 | H | 4,5 | N | 16,7 % |

b)

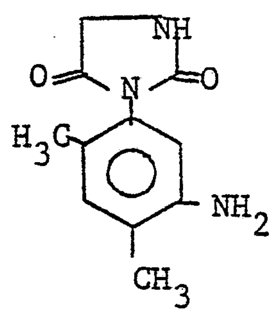

Obiges Zwischenprodukt (17,3 g, 0,0694 Mol) wurde in 170 ml Methylcellosolve mit 2 g Raney-Nickel hydriert. Aus der filtrierten und eingeengten Lösung kristallisierten 12,5 g (82,2%) 3-(4',6'-Dimethyl-3'-aminophenyl)-imidazolidin-2,4-dion, Smp. 187-188°C.

$C_{11} H_{13} N_3 O_2$ [219,24]

| | | | | | |
|---|---|---|---|---|---|
| Ber. C | 60,26 | H | 5,98 | N | 19,17 % |
| Gef. C | 60,2 | H | 6,1 | N | 19,0 % |

c)

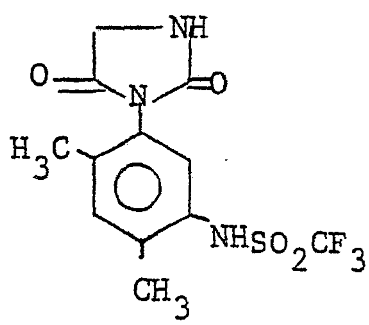

6,3 g (0,02876 Mol) des oben erhaltenen Zwischenprodukts und 3,65 g N,N-Dimethylanilin in 100 ml Chloroform (abs.) wurden mit 8,5 g $(CF_3SO_2)_2O$ (1,05 Aequ.) bei -5°C zur Reaktion gebracht (tropfenweises Addieren). Nach 3-stündigem Rühren bei Raumtemperatur wurde die Mischung mit 1N NaOH extrahiert. Letztere wurde filtriert, sauer gestellt (conc. HCl) und vom gefällten Produkt befreit. Dieses wurde gewaschen ($H_2O$) und getrocknet (70°C). Es verblieben 2,4 g (24%) 3-(4',6'-Dimethyl-3'-trifluormethansulfonamidophenyl)-imidazolidin-2,4-dion, Smp. 184-187°C.

$C_{12}H_{12}F_3N_3O_{11}S$ [351,30]

| | | | | | | | | | | |
|---|---|---|---|---|---|---|---|---|---|---|
| Ber. | C | 41,03 | H | 3,44 | N | 11,96 | S | 9,13 | F | 16,22% |
| Gef. | C | 38,6 | H | 3,8 | N | 11,2 | S | 8,9 | F | 14,5% |

Beispiel 4

Herstellung von 3-(4',6'-Dimethyl-3'-trifluormethansulfonylamido-phenyl)-1-methyl-imidazolidin-2,4-dion

[Typus Formel (5)]

a) 22 g NaOH und 52,5 g Sarcosin wurden in 300 ml Wasser gelöst:
101 g 4,6-Dimethyl-3-nitro-phenylisocyanat gelöst in 100 ml Essigester wurden bei 0-5°C unter Rühren zugetropft; der Ansatz wurde 2 Std. bei Raumtemperatur nachgerührt und dann die wässerige Phase abgetrennt und mit Salzsäure sauer gestellt: dabei fällt 1-Carboxymethyl-1-methyl-3-(4',6'-dimethyl-3'-nitro-phenyl)-harnstoff aus: Fp. 141-143° (Zers.)

b) Das nutschenfeuchte Produkt wurde mit 150 ml Eisessig und 150 ml Salzsäure 3 Std. bei 100° gerührt, nach Erkalten wurde in Eiswasser eingerührt und der Niederschlag von 1-(4',6'-Dimethyl-3'-nitro-phenyl)-3-methylhydantoin abgenutscht. Fp. 133-135°

c) Das Produkt wurde in der üblichen Weise mit Raney-Nickel in äthanolischer Lösung hydriert und 1-(3'-amino-4',6'-dimethyl-phenyl)-3-methyl-hydantoin vom Fp. 182-184° erhalten.

d) 23 g dieser Verbindung wurden mit 12 g N,N-Dimethylanilin in 100 ml Methylenchlorid vorgelegt und bei -30°, 28 g Trifluormethansulfonsäure-anhydrid zugetropft, die dunkle Lösung wurde 24 Std. bei Raumtemperatur stehengelassen, 1 Std. bei 50° gerührt und dann zu einer Lösung von 8 g NaOH in 300 ml $H_2O$ gegeben, die wässrige Phase wurde abgetrennt, mit A-Kohle geklärt und mit HCl sauer gestellt, dabei scheidet sich die Titelverbindung in kristalliner Form ab. Nach dem Trocknen Fp. 217-220°, Formel:

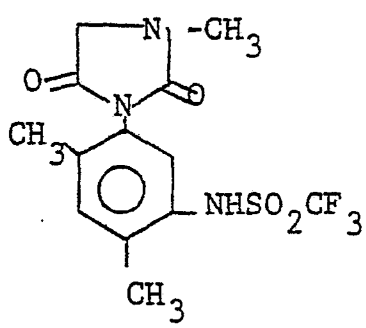

Diese Verbindung kann auch mit 1-(4',6'-Dimethyl-3'-trifluormethansulfonamidophenyl)-3-methylhydantoin bezeichnet werden.

Beispiel 5

Herstellung von 1-(4',6'-Dimethyl-3'-trifluormethan-sulfonamido -phenyl -3-methyl-imidazolin-2-on

[Typus Formel (8)]

a) 52 g 1-(4',6'-Dimethyl-3'-nitro-phenyl)-3-methyl-hydantoin (Beispiel 4b) wurden in 200 ml Methanol suspendiert und portionenweise bei 0° 7,5 g $NaBH_4$ eingetragen, der Ansatz wurde 2 Std. bei Raumtemperatur gerührt und dann mit Wasser verdünnt, das ausgefallene 1-(4',6'-Dimethyl-3'-nitro-phenyl)-3-methyl-5-hydroxy-imidazolidin-2-on wurde abgenutscht. Fp. 151-153°.

b) 53 g dieser Verbindung wurden in 250 ml Toluol unter Zusatz von 0,5 g Toluol-4-sulfonsäure 2 Std. unter Rückfluss erhitzt, das so entstehende Wasser wurde ausgekreist, nach Erkalten wurde die Lösung klarfiltriert, mit Wasser gewaschen und das Lösungsmittel abdestilliert, der Rückstand besteht aus 1-(4',6'-dimethyl-3'-nitro-phenyl)-3-methyl-imidazolin-2-on. Fp. 104-109°

c) Diese Verbindung wurde in üblicher Weise mit Raney-Nickel in methanolischer Lösung hydriert und 1-(3-Amino-4,6-dimethyl-phenyl-)3-methyl-imidazolin-2-on erhalten. Fp. 149-151°

d) 21 g dieser Verbindung wurden mit 12 g N,N-Dimethylanilin in 100 ml Methylenchlorid vorgelegt und bei -30° 23 g Trifluormethan-sulfonsäure-anhydrid zugetropft; der Ansatz wurde 24 Std. bei Raumremperatur stehengelassen, 1 Std. bei 50° gerührt und dann zu einer Lösung von 8 g NaOH in 300 ml Wasser gegeben, die wässerige Phase wurde abgetrennt, mit A-Kohle geklärt und mit HCl sauer gestellt, die Titelverbindung scheidet sich kristallin ab nach dem Trocknen. Fp. 213-216° Formel:

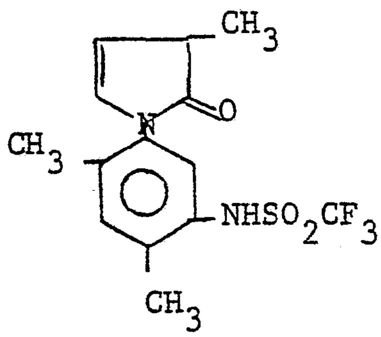

Beispiel 6

3-(4',6'-Dimethyl-3'-trifluormethansulfonamidophenyl)-oxazolidin-2-on

[Typus Formel (27)[

a)

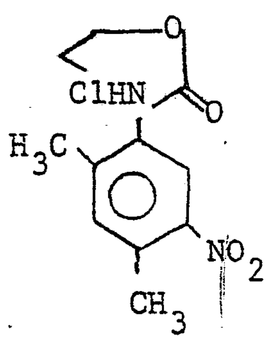

16,2 g (0,0845 Mol) 3-Nitro-4,6-dimethyl-phenylisocyanat (Bull. Soc. Chim. France 21, 952 (1899.) und 20 g 2-Chloräthyl-alkohol (Ueberschuss) in 150 ml Acetonitril werden während 18 Std. am Rückfluss gekocht. Man dampft dann die Reaktionsmischung ein und kristallisiert den festen Rückstand aus Aether um: man erhält 18 g 2-Chloräthyl-N-(3'-Nitro-4',6'-dimethyl-phenyl)-carbamat (78%), Smp. 133°

$C_{11} H_{13} Cl N_2 O_4$ [272,69]

| | | | | | | | | |
|---|---|---|---|---|---|---|---|---|
| Ber. | C | 48,45 | H | 4,81 | N | 10,27 | Cl | 31,00% |
| Gef. | C | 48,7 | H | 4,8 | N | 10,3 | Cl | 12,8% |

b) 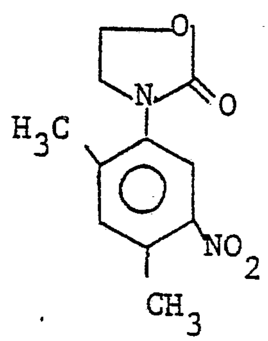

13,9 g (0,051 Mol) Chloräthyl-carbamat in 100 ml 15 proz. Natronlauge und 10 ml Tetrahydrofuran werden während 2 Std. bei 30-35° gerührt. Unter Eiskühlung stellt man mit konz. Salzsäure sauer, filtriert das Produkt ab, wäscht mit Wasser und Aether und trocknet bei 40° im Exsikkator: 10,4 g (86%) 3-(4',6'-Dimethyl-3'-nitrophenyl)-oxazolidin-2-on, Smp. 124-125°C

$C_{11} H_{12} N_2 O_4$ [236,23]

| | | | | | | |
|---|---|---|---|---|---|---|
| Ber. | C | 55,93 | H | 5,12 | N | 11,86% |
| Gef. | C | 54,7 | H | 5,1 | N | 11,5% |

c)

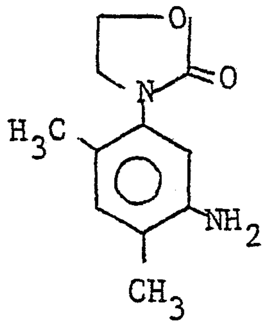

12,6 g (0,0534 Mol) Nitrophenyloxazolidin werden in 130 ml abs. Methanol mit 2,6 g Raney-Nickel bei 35°C hydriert. Die filtrierte Lösung wurde eingeengt, worauf Kristallisation des Produktes eintrat: 10,7 g (97%) 3-(4',6'-Dimethyl-3'-amino-phenyl)-oxazolidin-2-on, Smp. 162-163°

$C_{11}$ $H_{14}$ $N_2$ $O_2$ [206,25]

| | | | | | | |
|---|---|---|---|---|---|---|
| Ber. | C | 64,06 | H | 6,84 | N | 13,58% |
| Gef. | C | 63,8 | H | 6,9 | N | 13,4% |

d)

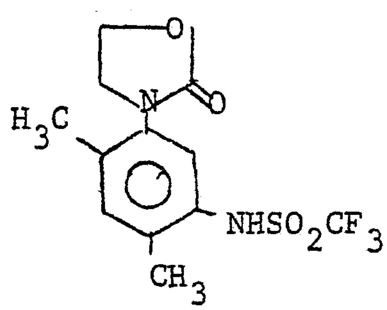

10,5 g Aminophenyloxazolidin (0,051 Mol) und 6,5 g N,N-Dimethylanilin (1,05 Aequ.) in 120 ml abs. Chloroform werden bei -5°C mit 15 g $(CF_3SO_2)_2O$ in 7 ml Chloroform (1,05 Aequ.) innert 20 Minuten versetzt. Man lässt 1 Std. ausrühren, wobei die Temperatur auf Raumtemperatur stieg. Die Reaktionslösung extrahiert man 2 mal mit je 250 ml 1N NaOH. Der basische Extrakt wurde filtriert und sauer gestellt (conc. HCl) und das gefällte Produkt abfiltriert. Nach dem Trocknen

(70°C) sammelt man 16,3 g (94,6%) 3-(4',6'-Dimethyl-3'-trifluormethansulfonamidophenyl)-oxazolidin-2-on, Smp. 207-209°.

$C_{12}$ $H_{13}$ $F_3$ $N_2$ $O_4$ S [338,30]

| | | | | | | | | | | |
|---|---|---|---|---|---|---|---|---|---|---|
| Ber. | C | 42,61 | H | 3,88 | N | 8,28 | S | 9,48 | F | 16,85% |
| Gef. | C | 42,1 | H | 3,9 | N | 7,9 | S | 9,4 | F | 16,8% |

Beispiel 7

Herstellung von 3-(4,6-Dimethyl-3-trifluormethansulfonyl-amino-phenyl)-5-oxazolidin-2,4-dion

[Typus Formel (29)]

a) 59 g L-(-)-Milchsäureäthylester und 96 g 4,6-Dimethyl-3-nitro-phenyl-isocyanat wurden in 200 ml Toluol gelöst, nach Zugabe von 0,5 ml Triäthylamin erwärmte sich die Lösung langsam und wurde 3 Std. bei 80° nachgerührt. Nach Einengen der Lösung und Verdünnen mit Hexan wurde O-(1-Carbäthoxy-äthyl)-N-(4,6-dimethyl-3-nitro-phenyl)-urethan vom Fp. 128-130° erhalten.

b) Diese Verbindung wurde durch 2 stündiges Rühren mit 150 ml konz. Salzsäure bei 90° zum 3-(4,6-Dimethyl-3-nitro-phenyl)-5-methyl-oxazolidin-2,4-dion cyclisiert. Fp. 123-125°

c) Diese Verbindung wurde in üblicher Weise mit Raney-Nickel in methanolischer Lösung hydriert und 3-(3-Amino-4,6-dimethyl-phenyl)-5-methyl-oxazolidin-2,4-dion vom Fp. 105-107° erhalten.

d) 13 g dieser Verbindung und 7 g N,N-Dimethyl-anilin wurden in 100 ml Methylenchlorid gelöst und 16 g Trifluormethansulfonsäure-anhydrid bei -30° zugetropft, die Lösung wurde 24 Std. bei Raumtemperatur stehengelassen, 1 Std. bei 50° gerührt und nach Erkalten mit einer Lösung von 5 g NaOH in 300 ml Wasser versetzt. Die wässerige Phase wurde abgetrennt, mit Aktivkohle geklärt und mit HCl sauer gestellt. Das abgeschiedene Oel wurde in Essigester aufgenommen, über $Na_2SO_4$ getrocknet und das Lösungsmittel abdestilliert, der Rückstand kristallisierte bei Verreiben mit Hexan und wenig Essigester. Die so erhaltene Titelverbindung zeigte einen Fp. 155-157° (Zers.)

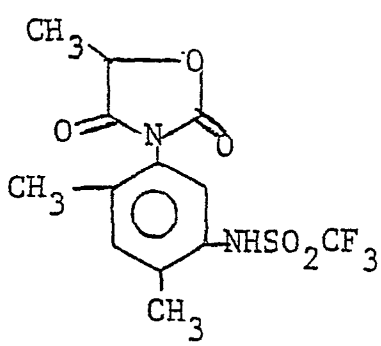

Beispiel 8

1-(3'-Trifluormethansulfonamido-4'-chlor-6'-methyl-phenyl)-1,3-sultam

[Typus Formel (48)]

Zu einer Lösung von 13 g (0,05 Mol) 1-(3'-Amino-4'-chlor-6'-methyl-phenyl)-1,3-sultam und 8,5 g (0,07 Mol) N,N-Dimethylanilin in 200 ml Chloroform werden bei 0°C 16,9 g (0,06 Mol) Trifluormethansulfonsäureanhydrid in 50 ml Chloroform innert 30 Min. zugetropft. Dann lässt man die Temperatur auf Raumtemperatur ansteigen. Die Lösung wird unter Eiszugabe dreimal mit 2N NaOH-Lösung extrahiert. Der wässerige Extrakt wird filtriert und mit konz. Salzsäure sauer gestellt. Das so gefällte Produkt wird abfiltriert, mit Wasser gewaschen, getrocknet und umkristallisiert. (Toluol)

Ausbaute: 14,6 g (75%); Smp. 150-151°C.

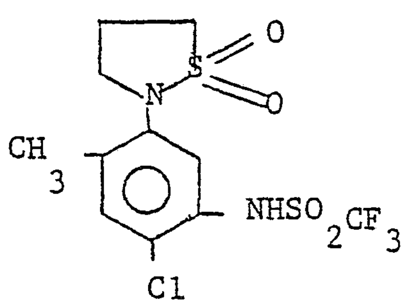

In den folgenden Tabellen ist der substituierte Phenylrest Ar in den Formeln mit $Ar_1$, $Ar_2$... etc. bis $Ar_9$ bezeichnet für welche Phenylreste die genaue Konstitution auf Seite 25 angegeben ist.

1-Aryl-3-alkyl-uretidin-2,4-dione der Formel (3)

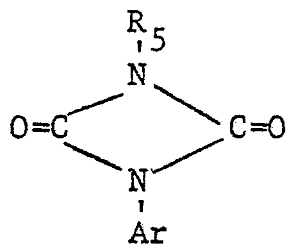

(3)

Herstellung in Analogie zu USP 3 579 500

| Ar | $R_5$ | physikal. Konstante |
|---|---|---|
| $Ar_1$ | $CH_3$ | |
| $Ar_2$ | $CH_3$ | |
| $Ar_3$ | $CH_3$ | |
| $Ar_4$ | $CH_3$ | |
| $Ar_5$ | $CH_3$ | |
| $Ar_6$ | $CH_3$ | |
| $Ar_7$ | $CH_3$ | |
| $Ar_1$ | $C_2H_5$ | |
| $Ar_2$ | $C_2H_5$ | |
| $Ar_3$ | $C_3H_7$ | |
| $Ar_4$ | $C_4H_9$ | |
| $Ar_5$ | $C_5H_{11}$ | |
| $Ar_6$ | $C_6H_{13}$ | |
| $Ar_7$ | $C_8H_{17}$ | |

1-Aryl-imidazolidin-2-one der Formel (4)

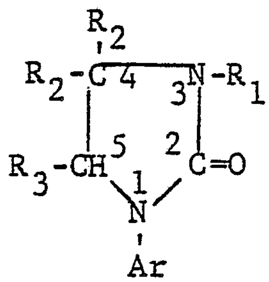

(4)

| Ar | $R_1$ | $R_2$ | $R_3$ | physikal. Daten |
|---|---|---|---|---|
| $Ar_2$ | $CH_3$ | H,H | $CH_3$ | Fp.183-185° ($CF_3SO_3H$-Salz) |
| $Ar_1$ | $i$-$C_3H_7$ | H,H | $CH_3$ | Fp. 176-180° |
| $Ar_2$ | Cyclopropyl | H,H | H | |
| $Ar_2$ | Cyclohexyl | H,H | H | Fp.179-185° ($CF_3SO_3H$-Salz) |
| $Ar_2$ | n-Hexyl | H,H | H | |
| $Ar_2$ | n-Octyl | H,H | H | |
| $Ar_2$ | 4-Chlorphenyl | H,H | H | |
| $Ar_2$ | 4-Methoxyphenyl | H,H | H | |
| $Ar_2$ | 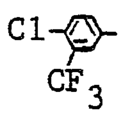 | H,H | H | |
| $Ar_1$ | Phenyl | H,H | H | |
| $Ar_1$ | H | $CH_3$,$CH_3$ | H | |
| $Ar_1$ | $CH_3$ | $CH_3$,$CH_3$ | H | |
| $Ar_2$ | $CH_3$ | H,H | H | Fp. 226-230° |
| $Ar_2$ | H | H,H | H | Fp. 288° |
| $Ar_3$ | N-Methylcarbamoyl | H,H | H | |
| $Ar_4$ | $CH_3$ | $CH_3$,$CH_3$ | H | |
| $Ar_2$ | $CH_3O$-$CH_2$- | H,H | H | |
| $Ar_6$ | $CH_3O$-$(CH_2)_2$- | H,H | H | |
| $Ar_7$ | $CH_3$ | H,Butyl | H | |

3-Aryl-imidazolidin-2,4-dione der Formel (5)

(3-Aryl-hydantoine)

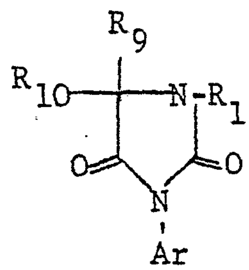

(5)

Herstellung in Analogie zu DAS 1 039 302 und USP 3 134 663.

| Ar | $R_1$ | $R_9$ | $R_{10}$ | physikal. Daten |
|---|---|---|---|---|
| $Ar_2$ | $CH_3$ | H | H | Fp. 217-220° |
| $Ar_2$ | $i-C_3H_7$ | H | H | Fp. 209-211° |
| $Ar_2$ | $t-C_4H_9$ | H | H | |
| $Ar_1$ | Cyclopropyl | H | H | Fp.165-168° (Zers.) |
| $Ar_2$ | Cyclohexyl | H | H | |
| $Ar_1$ | H | $CH_3$ | $CH_3$ | Fp.139-142° (Zers.) |
| $Ar_1$ | 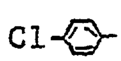 | H | H | |
| $Ar_2$ | 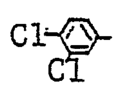 | H | H | |
| $Ar_1$ | 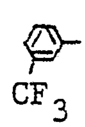 | H | H | Fp.201-205° |
| $Ar_2$ | H | H | H | Fp. 184-187° |
| $Ar_5$ | H | Tetramethylen | | |
| $Ar_3$ | N-Methyl-carbamoyl | H | H | |
| $Ar_4$ | N-Phenyl-carbamoyl | H | H | |
| $Ar_6$ | Carbäthoxy | H | H | |
| $Ar_7$ | Acetyl | H | H | |
| $Ar_2$ | 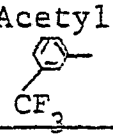 | H | H | Fp. > 250° |

1-Aryl-imidazolidin-2,4-dione der Formel (6)
(1-Aryl-hydantoine)

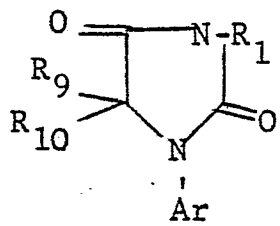

(6)

| Ar | $R_1$ | $R_9$ | $R_{10}$ | physikal. Daten |
|---|---|---|---|---|
| $Ar_1$ | $CH_3$ | H | H | Fp.199-200° |
| $Ar_1$ | tert.$C_4H_9$ | H | H | Fp.194-197° (Zers.) |

1-Aryl-imidazolidin-2,4,5-trione der Formel (7)

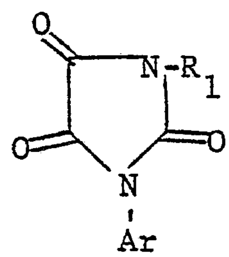

(7)

Die Herstellung der zu reduzierenden und dann mit $(CF_3SO_2)_2O$ zu behandelnden Nitrophenyl-Ausgangsverbindung geschieht in Analogie zu USP 3 481 334 und DOS 2 214 448 nach folgendem Reaktionsschema:

$O_2N-Ar-NH_2 + O=C=N-R_1$
oder
$O_2N-Ar-N=C=O + H_2N-R_1$

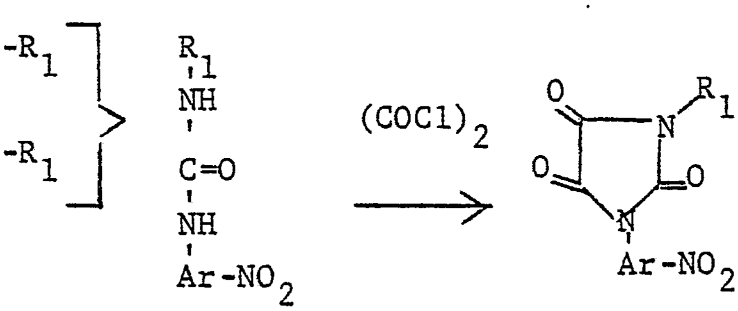

| Ar | $R_1$ | Fp | Ar | $R_1$ | Fp |
|---|---|---|---|---|---|
| $Ar_7$ | $-C_6H_5$ | 185-88° | $Ar_2$ | $-COOCH_3$ | |
| $Ar_5$ | $-CH_3$ | | $Ar_2$ | -COO-(H) | |
| $Ar_2$ | $-C_2H_5$ | 171-72° | $Ar_3$ | $-COOCH_3$ | |
| $Ar_1$ | $i-C_3H_7$ | | $Ar_3$ | -COO-Benzyl | |
| $Ar_3$ | $-C_2H_5$ | | $Ar_4$ | $-COOCH_3$ | |
| $Ar_4$ | $-CH_3$ | | $Ar_4$ | $-COOCH_2-CH=CH_2$ | |
| $Ar_6$ | $n-C_3H_7$ | | $Ar_5$ | $-COOCH_3$ | |
| $Ar_1$ | $CH_3$ | | $Ar_5$ | $-COO-CH_2CH_2Br$ | |
| $Ar_1$ | $C_2H_5$ | | $Ar_6$ | $-COOCH_3$ | |
| $Ar_2$ | $CH_3$ | | $Ar_7$ | $-COOC_2H_5$ | |
| $Ar_2$ | $C_6H_5$ | | | | |
| $Ar_3$ | $CH_3$ | | | | |
| $Ar_1$ | $-COOCH_3$ | | | | |
| $Ar_1$ | $-COOC_2H_5$ | | | | |

1-Aryl-imidazolin-2-one der Formel (8)

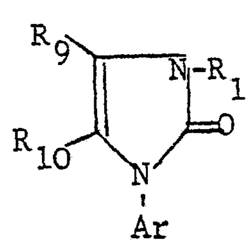

(8)

Herstellung entsprechend Ber. 31, 2129 (1898); J.Chem.Soc. 101, 2352 (1912)

Ber. 40, 4666 (1907); J.Chem.Soc. 103,56 (1913)

Ber. 43, 2796 (1910)

| Ar | $R_1$ | $R_9$ | $R_{10}$ | Fp. |
|---|---|---|---|---|
| $Ar_2$ | $CH_3$ | H | H | 213-216° |
| $Ar_1$ | $i\text{-}C_3H_7$ | H | H | 184-187° |
| $Ar_1$ | 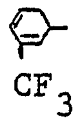 | H | H | |
| $Ar_2$ | $-C_2H_5$ | H | $CH_3$ | 236-240° |
| $Ar_2$ | 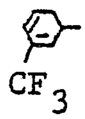 | H | $CH_3$ | |
| $Ar_1$ | $i\text{-}C_3H_7$ | H | $CH_3$ | 226-229° |
| $Ar_2$ | $CH_3$ | $CH_3$ | H | 181-185° |
| $Ar_2$ | 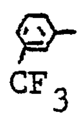 | $C_2H_5$ | H | |

<u>1-Aryl-imidazole der Formel (9)</u>

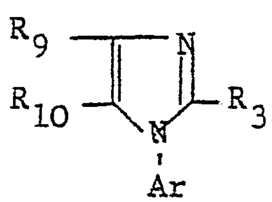

(9)

Die Herstellung erfolgt gemäss L.A. Paquette, "Principles of Modern Heterocyclic Chemistry", Seite 194 bezw. J.A.C.S. <u>71</u>, 383 (1949) nach folgendem Formelschema:

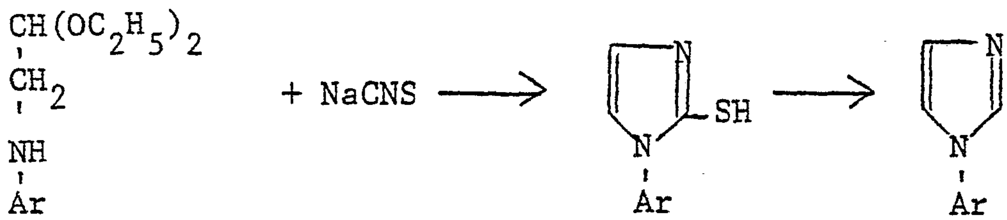


oder

durch Umsetzung von Imidazolen mit reaktiven Arylhalogeniden in Gegenwart einer Base.

| Ar | $R_3$ | $R_9$ | $R_{10}$ | Fp. |
|---|---|---|---|---|
| $Ar_1$ | H | H | H | |
| $Ar_2$ | H | H | H | |
| $Ar_3$ | H | H | H | |
| $Ar_4$ | H | H | H | |
| $Ar_5$ | H | H | H | |
| $Ar_6$ | H | H | H | |
| $Ar_7$ | H | H | H | |
| $Ar_1$ | $-SC_2H_2$ | H | H | |
| $Ar_2$ | $-S-C_3H_7(n)$ | H | H | |
| $Ar_3$ | $-S-C_3H_7(i)$ | H | H | |
| $Ar_4$ | $-S-C_4H_9(t)$ | H | H | |

ferner die Verbindungen der Formeln

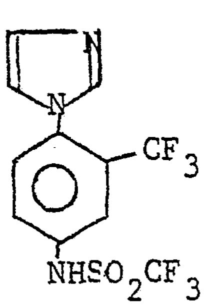

und

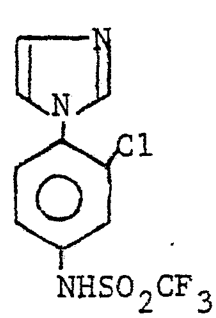

Von den 1-Aryl-imidazolin-4-onen der Formel (10) sei die Verbindung

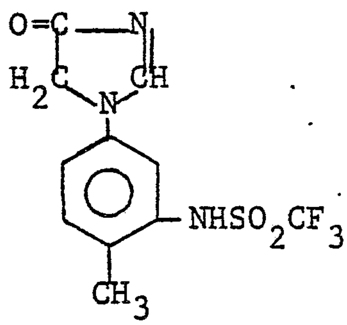

1-(3'-Trifluormethansulfonamido-4'-methyl-phenyl)-imidazolin-4-on.

als Verteter erwähnt.

1-Aryl-1,4,5,6-tetrahydro-pyrimidin-2-one der Formel (11)

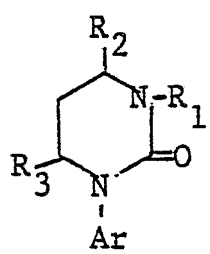

(11)

| Ar | $R_1$ | $R_2$ | $R_3$ | Fp. |
|---|---|---|---|---|
| $Ar_2$ | $i-C_3H_7$ | H | H | |
| $Ar_7$ | $i-C_4H_9$ | H | H | |
| $Ar_1$ | $CH_3$ | H | H | |
| $Ar_3$ | $CH_3$ | $CH_3$ | H | |

1-Aryl-hexahydro-pyrimidine der Formel (12)

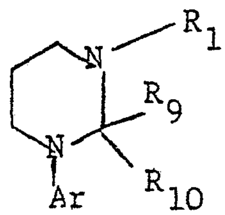

| Ar | $R_1$ | $R_9$ | $R_{10}$ | Fp. |
|---|---|---|---|---|
| $Ar_1$ | $CH_3$ | H | H | |
| $Ar_2$ | $CH_3$ | H | H | |
| $Ar_3$ | $C_2H_5$ | Benzyl | H | |
| $Ar_2$ | $CH_3$ | $CH_3$ | H | |
| $Ar_2$ | $-CO-CH_3$ | H | H | |
| $Ar_3$ | -CO-NH-Phenyl | H | H | |
| $Ar_5$ | $-CO-O-C_2H_5$ | H | H | |

Herstellung analog zu A. Weissberger, The Chemistry of Heterocyclic Compounds" Vol. 16, S. 453 (J.Wiley, 1962).

1-Aryl-pyrimidin-2-one der Formel (13)

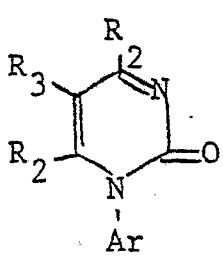

Herstellung nach A. Weissberger (loc.cit.) Vol. 16, S. 357.

| Ar | $R_2$ | $R_3$ | Fp. |
|---|---|---|---|
| $Ar_1$ | H,H | H | |
| $Ar_2$ | H,H | H | |
| $Ar_3$ | $CH_3$,$CH_3$ | H | |
| $Ar_2$ | $CH_3$,$CH_3$ | H | |
| $Ar_4$ | H,H | $CH_3$ | |
| $Ar_5$ | H,H | CN | |

1-Aryl-1,4-dihydro-pyrimidin-4-one der Formel (14)

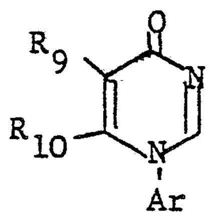

| Ar | $R_9$ | $R_{10}$ | Fp. |
|---|---|---|---|
| $Ar_1$ | H | H | |
| $Ar_2$ | H | H | |
| $Ar_3$ | H | H | |
| $Ar_4$ | $CH_3$ | H | |
| $Ar_7$ | H | $CH_3$ | |

Herstellung nach A. Weissberger (loc.cit.) Vol. 16, S. 359.

1-Aryl-uracile der Formel (15)

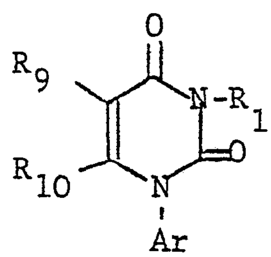

Herstellung analog zu DOS 2 126 148

| Ar | $R_1$ | $R_9$ | $R_{10}$ | Fp. |
|---|---|---|---|---|
| $Ar_1$ | H | H | H | |
| $Ar_2$ | H | H | H | |
| $Ar_2$ | $CH_3$ | H | H | |
| $Ar_3$ | $CH_3$ | Phenyl | H | |
| $Ar_1$ | $i-C_3H_7$ | $CH_3$ | H | |
| $Ar_2$ | $C_2H_5$ | $CH_3$ | Cl | |
| $Ar_5$ | $CH_3$ | H | $-OCH_3$ | |
| $Ar_1$ | $CH_3$ | H | $CH_3$ | |
| $Ar_2$ | $CH_3$ | $CH_3$ | $-N(CH_3)_2$ | |

3-Aryl-uracile der Formel (16)

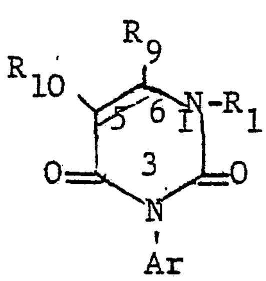

Herstellung analog USP 3 254 082

| Ar | $R_1$ | $R_9$ | $R_{10}$ | Fp. |
|---|---|---|---|---|
| $Ar_1$ | H | $CH_3$ | H | |
| $Ar_2$ | H | H | $CH_3$ | |
| $Ar_2$ | $CH_3$ | H | $CH_3$ | |
| $Ar_1$ | $CH_3$ | $CH_3$ | $CH_3$ | |

<u>1-Aryl-5,6-dihydro-uracile der Formel (17)</u>

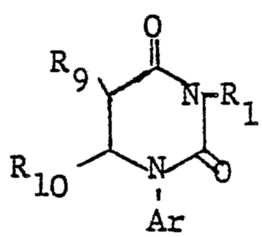

Herstellung analog zu Weissberger (loc.cit.), Bd. 16, S. 437

| Ar | $R_1$ | $R_9$ | $R_{10}$ | Fp. |
|---|---|---|---|---|
| $Ar_1$ | H | $CH_3$ | H | |
| $Ar_3$ | $CH_3$ | H | $CH_3$ | |
| $Ar_2$ | H | H | H | |
| $Ar_2$ | $CH_3$ | H | H | |

1-Aryl-4,5-dihydro-uracile der Formel (18)

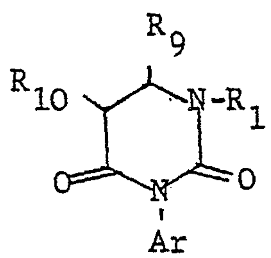

Herstellung analog Ber. 34, 3751 (1901)
J.A.C.S. 52, 4993 (1930)
J.A.C.S. 58, 299 (1936)
J.A.C.S. 69, 1382 (1947)

| Ar | $R_1$ | $R_9$ | $R_{10}$ | Fp. |
|---|---|---|---|---|
| $Ar_1$ | $C_2H_5$ | H | H | 251 - 252° |
| $Ar_2$ | $i-C_3H_7$ | H | H | |
| $Ar_2$ | $C_4H_9$ | H | H | |
| $Ar_1$ | Cyclohexyl | H | H | 228 - 230° |
| $Ar_4$ | H | H | H | |
| $Ar_5$ | $-CO-CH_3$ | H | H | |
| $Ar_3$ | $CH_3$ | H | $CH_3$ | |

1-Aryl-piperazin-2,6-dione der Formel (19)

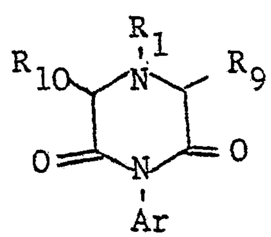

Herstellung analog zu: Ber. 52, 225 (1919)
Ber. 22, 1795 (1889)
Ber. 23, 1987 (1890)

| Ar | $R_1$ | $R_9$ | $R_{10}$ | Fp. |
|---|---|---|---|---|
| $Ar_1$ | $i-C_3H_7$ | H | H | |
| $Ar_1$ | $CH_3$ | $CH_3$ | H | |

1-Aryl-piperazin-2,5-dione der Formel (20)

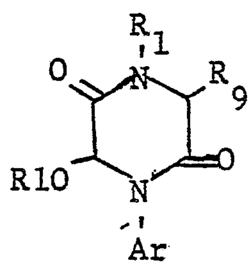

Herstellung analog zu Ber. 36, 2113 (1903)
Ber. 42, 4471 (1909)

| Ar | $R_1$ | $R_9$ | $R_{10}$ | Fp. |
|---|---|---|---|---|
| $Ar_1$ | $CH_3$ | H | H | |
| $Ar_1$ | $CH_3$ | $CH_3$ | $CH_3$ | |

1-Aryl-piperazin-2,3-dione der Formel (21)

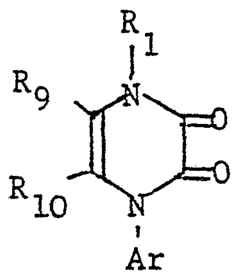

| Ar | $R_1$ | $R_9$ | $R_{10}$ | Fp. |
|---|---|---|---|---|
| $Ar_1$ | H | H | H | |
| $Ar_2$ | $CH_3$ | H | H | |

1-Aryl-dehydro-piperazin-2-one der Formel (22)

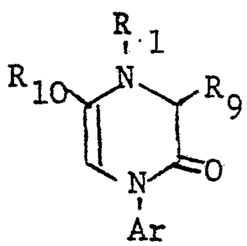

Darstellung nach "Elderfield" (loc.cit.) Vol. 6

| Ar | $R_1$ | $R_9$ | $R_{10}$ | Fp. |
|---|---|---|---|---|
| $Ar_1$ | $C_2H_5$ | H | H | |
| $Ar_1$ | $C_2H_5$ | $CH_3$ | H | |
| $Ar_1$ | $C_2H_5$ | H | $CH_3$ | |
| $Ar_2$ | $CH_3$ | H | H | |
| $Ar_2$ | $CH_3$ | $CH_3$ | H | |
| $Ar_2$ | $C_2H_5$ | H | H | |
| $Ar_2$ | $i\text{-}C_3H_7$ | H | H | |
| $Ar_3$ | $C_2H_5$ | H | H | |

| Ar | $R_1$ | $R_9$ | $R_{10}$ | Fp. |
|---|---|---|---|---|
| $Ar_3$ | $C_2H_5$ | $CH_3$ | H | |
| $Ar_3$ | $C_2H_5$ | H | $CH_3$ | |
| $Ar_4$ | $CH_3$ | H | H | |
| $Ar_4$ | $CH_3$ | $CH_3$ | H | |
| $Ar_4$ | $CH_3$ | H | $CH_3$ | |
| $Ar_5$ | $C_2H_5$ | H | H | |
| $Ar_5$ | $C_2H_5$ | H | $CH_3$ | |
| $Ar_5$ | $i\text{-}C_3H_7$ | H | H | |
| $Ar_6$ | $C_2H_5$ | H | H | |
| $Ar_6$ | $C_2H_5$ | $CH_3$ | H | |
| $Ar_6$ | $C_2H_5$ | H | $CH_3$ | |
| $Ar_7$ | $C_2H_5$ | H | H | |
| $Ar_7$ | $C_2H_5$ | $CH_3$ | H | |
| $Ar_7$ | $C_2H_5$ | H | $CH_3$ | |
| $Ar_7$ | $C_6H_5$ | H | H | |

1-Aryl-piperazin-2,3,5-trione der Formel (23)

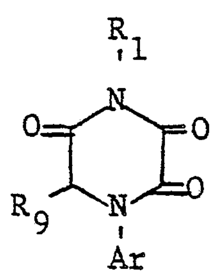

Herstellt nach Elderfield, Vol. 6 nach dem Schema:

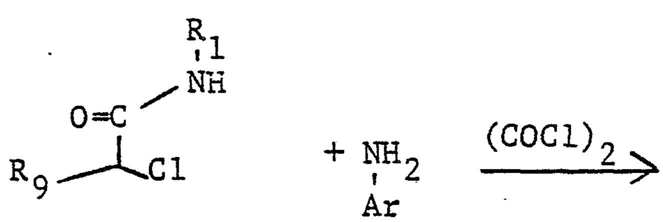


(23)

| Ar | $R_1$ | $R_9$ | Fp: |
|---|---|---|---|
| $Ar_1$ | $CH_3$ | H | |
| $Ar_1$ | $C_2H_5$ | $CH_3$ | |
| $Ar_1$ | $i\text{-}C_3H_7$ | H | |
| $Ar_1$ | Benzyl | H | |
| $Ar_1$ | Phenyl | H | |
| $Ar_2$ | $C_2H_5$ | H | |
| $Ar_2$ | $CH_3$ | $CH_3$ | |
| $Ar_2$ | $i\text{-}C_3H_7$ | H | |
| $Ar_3$ | $C_2H_5$ | H | |
| $Ar_3$ | $CH_3$ | $CH_3$ | |
| $Ar_3$ | $i\text{-}C_3H_7$ | H | |
| $Ar_4$ | $C_2H_5$ | H | |
| $Ar_4$ | $CH_3$ | $CH_3$ | |
| $Ar_4$ | $i\text{-}C_3H_7$ | H | |
| $Ar_5$ | $C_2H_5$ | H | |
| $Ar_5$ | $CH_3$ | $CH_3$ | |
| $Ar_5$ | $i\text{-}C_3H_7$ | H | |
| $Ar_6$ | $C_2H_5$ | H | |
| $Ar_6$ | $CH_3$ | $CH_3$ | |
| $Ar_6$ | $i\text{-}C_3H_7$ | H | |
| $Ar_7$ | $C_2H_5$ | H | |
| $Ar_7$ | $CH_3$ | $CH_3$ | |
| $Ar_7$ | $i\text{-}C_3H_7$ | H | |

1-Aryl-piperazin-2,3,6-trione der Formel (24)

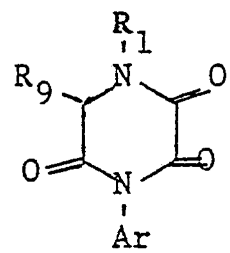

(24)

Herstellung gemäss Elderfield, Vol. 6 nach dem Schema:

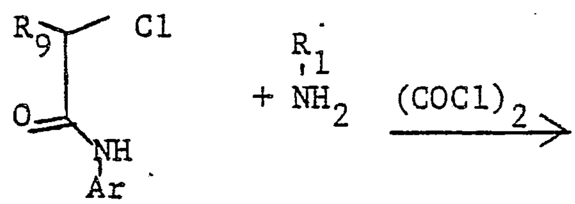


(24)

| Ar | $R_1$ | $R_9$ | Fp. |
|---|---|---|---|
| $Ar_1$ | $CH_3$ | H | |
| $Ar_1$ | $C_2H_5$ | $CH_3$ | |
| $Ar_1$ | $i-C_3H_7$ | H | |
| $Ar_1$ | Benzyl | H | |
| $Ar_1$ | Phenyl | H | |
| $Ar_2$ | $C_2H_5$ | H | |
| $Ar_2$ | $CH_3$ | $CH_3$ | |
| $Ar_2$ | $i-C_3H_7$ | H | |
| $Ar_3$ | $C_2H_5$ | H | |
| $Ar_3$ | $CH_3$ | $CH_3$ | |
| $Ar_3$ | $i-C_3H_7$ | H | |
| $Ar_4$ | $C_2H_5$ | H | |
| $Ar_4$ | $CH_3$ | $CH_3$ | |
| $Ar_4$ | $i-C_3H_7$ | H | |
| $Ar_5$ | $C_2H_5$ | H | |
| $Ar_5$ | $CH_3$ | $CH_3$ | |
| $Ar_5$ | $i-C_3H_7$ | H | |

| Ar | $R_1$ | $R_9$ | Fp. |
|---|---|---|---|
| $Ar_6$ | $C_2H_5$ | H | |
| $Ar_6$ | $CH_3$ | $CH_3$ | |
| $Ar_6$ | $i-C_3H_7$ | H | |
| $Ar_7$ | $C_2H_5$ | H | |
| $Ar_7$ | $CH_3$ | $CH_3$ | |
| $Ar_7$ | $i-C_3H_7$ | H | |

1-Aryl-piperazin-2,3,5,6-tetrone der Formel (25)

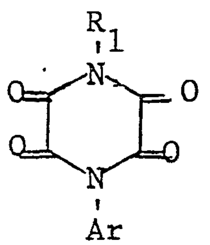

Herstellung nach Elderfield, Vol.6.

| Ar | $R_1$ | Fp. |
|---|---|---|
| $Ar_1$ | $CH_3$ | |
| $Ar_1$ | $C_2H_5$ | |
| $Ar_1$ | $i-C_3H_7$ | |
| $Ar_2$ | $CH_3$ | |
| $Ar_2$ | $C_2H_5$ | |
| $Ar_2$ | $i-C_3H_7$ | |
| $Ar_3$ | $CH_3$ | |
| $Ar_3$ | $C_2H_5$ | |
| $Ar_3$ | $i-C_3H_7$ | |
| $Ar_4$ | $CH_3$ | |
| $Ar_4$ | $C_2H_5$ | |

| Ar | $R_1$ | Fp. |
|---|---|---|
| $Ar_4$ | $i\text{-}C_3H_7$ | |
| $Ar_5$ | $CH_3$ | |
| $Ar_5$ | $C_2H_5$ | |
| $Ar_5$ | $i\text{-}C_3H_7$ | |
| $Ar_6$ | $CH_3$ | |
| $Ar_6$ | $C_2H_5$ | |
| $Ar_6$ | $i\text{-}C_3H_7$ | |
| $Ar_7$ | $CH_3$ | |
| $Ar_7$ | $C_2H_5$ | |
| $Ar_7$ | $i\text{-}C_3H_7$ | |
| $Ar_7$ | p-Chlorphenyl | |

3-Aryl-oxazolidin-2-one der Formel (27)

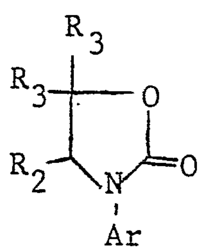

Herstellung analog zu R.C. Elderfield, "Heterocyclic compounds", Vol. 5, S. 396.

| Ar | $R_2$ | $R_3$ | Fp. |
|---|---|---|---|
| $Ar_1$ | H | H,H | |
| $Ar_2$ | H | H,H | |
| $Ar_3$ | H | H,H | |
| $Ar_4$ | H | H,H | |
| $Ar_5$ | H | H,H | |
| $Ar_7$ | H | H,H | |
| $Ar_6$ | H | $CH_3$,H | |
| $Ar_1$ | H | CN,H | |
| $Ar_2$ | $C_2H_5$ | H,H | |
| $Ar_1$ | H | $CH_3$, $CH_3$ | |
| $Ar_2$ | H | Phenyl, H | |
| $Ar_2$ | $C_8H_{17}$ | H,H | |
| $Ar_1$ | H | $-CH_2-OCH_3$,H | |

3-Aryl-oxazolidine der Formel (28)

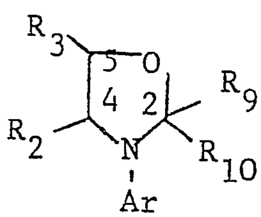

Herstellung analog DOS 2 218 097

| Ar | $R_2$ | $R_3$ | $R_9$ | $R_{10}$ | Fp. |
|---|---|---|---|---|---|
| $Ar_1$ | H | H | H | H | |
| $Ar_2$ | H | H | H | H | |
| $Ar_3$ | H | $CH_3$ | H | H | |
| $Ar_4$ | H | H | $CH_3$ | H | |
| $Ar_1$ | H | H | $CH_3$ | $CH_3$ | |

| Ar | $R_2$ | $R_3$ | $R_9$ | $R_{10}$ | Fp. |
|---|---|---|---|---|---|
| $Ar_2$ | H | H | $(CH_2)_4$ | | |
| $Ar_2$ | H | H | $(CH_2)_5$ | | |
| $Ar_5$ | $CH_3$ | $CH_3$ | $CH_3$ | H | |
| $Ar_1$ | H | H | $C_3H_7$ | H | |

3-Aryl-oxazolidin-2,4-dione der Formel (29)

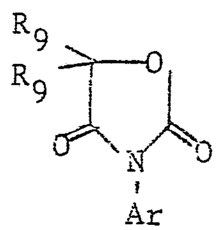

Herstellung gemäss Bull.Soc.chim.France[3] 29, 122 (1903)
27, 444 (1902)
19, 780 (1898)

| Ar | $R_9$ | Fp. |
|---|---|---|
| $Ar_1$ | $CH_3$,H | |
| $Ar_2$ | $CH_3$,H | 155-157° |
| $Ar_1$ | $CH_3$, $CH_3$ | |
| $Ar_2$ | H,H | |
| $Ar_3$ | $CH_3$, H | |
| $Ar_4$ | $CH_3$, $CH_3$ | |
| $Ar_5$ | $C_2H_5$, H | |
| $Ar_6$ | H, H | |

3-Aryl-thiazolidin-2-one der Formel (30)

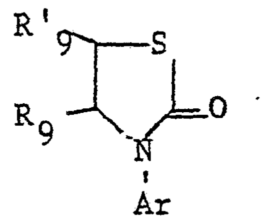

Herstellung nach R.C. Elderfield, Vol. 5, S. 484

| Ar | $R_9$ | $R'_9$ | Fp. |
|---|---|---|---|
| $Ar_2$ | H | H | |
| $Ar_1$ | $CH_3$ | H | |

3-Aryl-thiazolidin-2,4-dione der Formel (31)

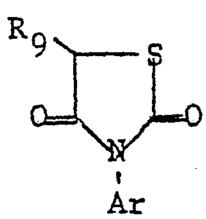

Herstellung nach R.C. Elderfield, Vol. 5, S. 711

| Ar | $R_9$ | Fp. |
|---|---|---|
| $Ar_2$ | H | |
| $Ar_6$ | $CH_3$ | |

3-Aryl-thiazolin-2-one der Formel (32)

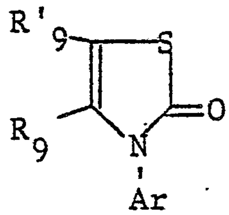

Herstellung nach R.C. Elderfield, Vol.5, S. 484

| Ar | $R_9$ | $R'_9$ | Fp. |
|---|---|---|---|
| $Ar_1$ | H | H | |
| $Ar_7$ | $CH_3$ | H | |
| $Ar_2$ | H | $CH_3$ | |

1-Aryl-4,5-dihydro-6(1H)-pyridazinone der Formel (34)

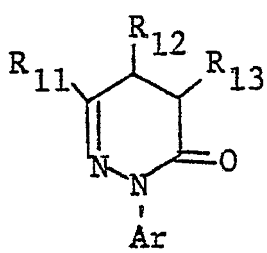

Herstellung A. Weissberger u.E. Taylor, "The Chemistry of Heterocyclic Compounds, 28, S. 26.

| Ar | $R_{11}$ | $R_{12}$ | $R_{13}$ | Fp. |
|---|---|---|---|---|
| $Ar_1$ | H | H | H | |
| $Ar_2$ | H | H | H | |
| $Ar_3$ | H | H | H | |
| $Ar_2$ | $CH_3$ | H | $CH_3$ | |
| $Ar_4$ | $CH_3$ | H | $CH_3$ | |
| $Ar_7$ | $CH_3$ | $CH_3$ | H | |
| $Ar_2$ | $CH_3$ | H | $i-C_3H_7$ | |
| $Ar_1$ | $-COOCH_3$ | H | H | |
| $Ar_1$ | -COOH | H | H | |
| $Ar_2$ | $-COOCH_3$ | H | H | |
| $Ar_2$ | -COOH | H | H | |
| $Ar_1$ | $CH_3$ | Phenyl | H | |
| $Ar_3$ | $-OCH_3$ | H | H | |

<u>1-Aryl-6(1H)-pyridazinone der Formel(34a)</u>

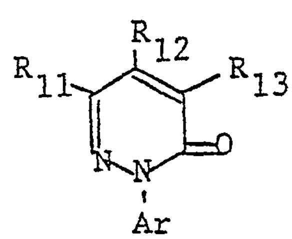

Herstellung nach Weissberger u. Taylor(siehe oben)

| Ar | $R_{11}$ | $R_{12}$ | $R_{13}$ | Fp. |
|---|---|---|---|---|
| $Ar_2$ | H | H | H | |
| $Ar_1$ | H | H | H | |
| $Ar_2$ | $-N(CH_3)_2$ | H | H | |
| $Ar_1$ | $-OCH_3$ | H | H | |
| $Ar_6$ | -COOH | H | H | |
| $Ar_6$ | $-COOCH_3$ | H | H | |
| $Ar_3$ | Cl | H | H | |
| $Ar_1$ | $CH_3$ | H | H | |
| $Ar_2$ | H | H | $CH_3$ | |
| $Ar_2$ | H | H | $-OCH_3$ | |
| $Ar_3$ | H | $-COOCH_3$ | H | |
| $Ar_1$ | H | $CH_3$ | H | |
| $Ar_2$ | Cl | Cl | H | |
| $Ar_2$ | $-COOCH_3$ | $CH_3$ | H | |
| $Ar_7$ | $CH_3$ | H | $CH_3$ | |
| $Ar_5$ | $CH_3$ | H | $-N(C_2H_5)_2$ | |
| $Ar_3$ | Phenyl | H | Cl | |
| $Ar_2$ | H | Cl | Cl | |
| $Ar_2$ | H | $-N(CH_3)_2$ | Cl | |
| $Ar_4$ | H | $-OCH_3$ | Cl | |
| $Ar_1$ | H | $-OCH_3$ | $-OCH_3$ | |

1-Aryl-pyridazin-3,6-dione der Formel (35)

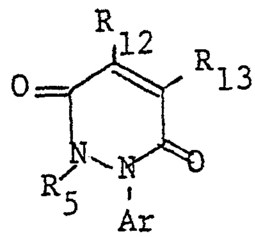

Herstellung analog zu Weissberger u. Taylor, 28, S. 28

| Ar | $R_5$ | $R_{12}$ | $R_{13}$ | Fp. |
|---|---|---|---|---|
| $Ar_2$ | $CH_3$ | H | H | |
| $Ar_2$ | $C_2H_5$ | H | H | |
| $Ar_5$ | $CH_3$ | $-N(CH_3)_2$ | H | |
| $Ar_1$ | $C_2H_5$ | $-OCH_3$ | H | |

1-Aryl-4,5-dihydro-pyridazin-3,6-dione der Formel (36)

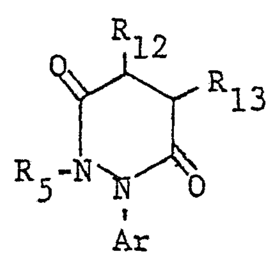

Herstellung nach Weissberger u. Taylor, 28, S. 50

| Ar | $R_5$ | $R_{12}$ | $R_{13}$ | Fp. |
|---|---|---|---|---|
| $Ar_1$ | $CH_3$ | H | H | |
| $Ar_2$ | $C_2H_5$ | H | H | |

1-Aryl-pyridazin-4-(1H)-one der Formel (37)

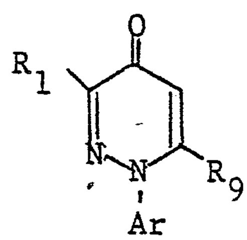

Herstellung nach Weissberger 28, S. 33.

| Ar | $R_1$ | $R_9$ | Fp. |
|---|---|---|---|
| $Ar_1$ | $-COOH$ | $CH_3$ | |
| $Ar_1$ | $-COOCH_3$ | $CH_3$ | |
| $Ar_2$ | $-COOH$ | $CH_3$ | |
| $Ar_2$ | $-COOCH_3$ | $CH_3$ | |
| $Ar_3$ | $-COOH$ | $CH_3$ | |
| $Ar_3$ | $-CO-N(CH_3)_2$ | $CH_3$ | |

1-Aryl-pyridazin-4,6-dione der Formel(38)

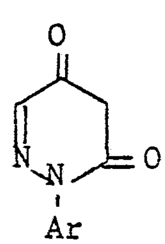

Herstellung nach Weissberger u. Taylor 28, S. 170

Beispiel: Ar = $Ar_1$

1-Aryl-pyrazole der Formel (39)

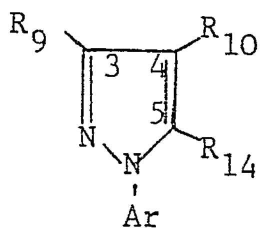

Herstellung nach Weissberger oder Houben-Weyl 10/2.

| Ar | $R_9$ | $R_{10}$ | $R_{14}$ | Fp. |
|---|---|---|---|---|
| $Ar_1$ | H | H | H | |
| $Ar_2$ | H | H | H | |
| $Ar_3$ | H | H | H | |
| $Ar_4$ | H | H | H | |
| $Ar_5$ | H | H | H | |
| $Ar_6$ | H | H | H | |
| $Ar_7$ | H | H | H | |
| $Ar_1$ | $-COOCH_3$ | $-COOCH_3$ | H | |
| $Ar_2$ | $-COOCH_3$ | $-COOCH_3$ | H | |
| $Ar_3$ | $-COOCH_3$ | $-COOCH_3$ | H | |
| $Ar_4$ | $-COOCH_3$ | $-COOCH_3$ | H | |
| $Ar_5$ | $-COOCH_3$ | $-COOCH_3$ | H | |
| $Ar_6$ | $-COOCH_3$ | $-COOCH_3$ | H | |
| $Ar_7$ | $-COOCH_3$ | $-COOCH_3$ | H | |
| $Ar_1$ | $CH_3$ | H | H | |
| $Ar_2$ | $CH_3$ | H | H | |
| $Ar_3$ | $CH_3$ | H | H | |
| $Ar_4$ | $CH_3$ | H | H | |
| $Ar_5$ | $CH_3$ | H | H | |
| $Ar_6$ | $CH_3$ | H | H | |
| $Ar_2$ | $CH_3$ | H | $-OCOCH_3$ | |

| Ar | $R_9$ | $R_{10}$ | $R_{14}$ | Fp. |
|---|---|---|---|---|
| $Ar_7$ | $CH_3$ | H | H | |
| $Ar_1$ | $CH_3$ | H | Cl | |
| $Ar_2$ | $CH_3$ | H | Cl | |
| $Ar_3$ | $CH_3$ | H | Cl | |
| $Ar_4$ | $CH_3$ | H | Cl | |
| $Ar_5$ | $CH_3$ | H | Cl | |
| $Ar_6$ | $CH_3$ | H | Cl | |
| $Ar_7$ | $CH_3$ | H | Cl | |
| $Ar_1$ | $-CCl_3$ | H | H | |
| $Ar_3$ | $-CCl_3$ | H | H | |
| $Ar_5$ | $-CCl_3$ | H | H | |
| $Ar_7$ | $-CCl_3$ | H | H | |
| $Ar_2$ | H | H | $CH_3$ | |
| $Ar_4$ | H | H | $CH_3$ | |
| $Ar_6$ | H | H | $CH_3$ | |
| $Ar_1$ | $i-C_3H_7$ | H | H | |
| $Ar_2$ | $i-C_3H_7$ | H | H | |
| $Ar_5$ | $i-C_3H_7$ | H | H | |
| $Ar_7$ | $i-C_3H_7$ | H | H | |
| $Ar_1$ | $CH_3$ | H | $CH_3$ | |
| $Ar_4$ | $CH_3$ | H | $CH_3$ | |
| $Ar_6$ | $CH_3$ | H | $CH_3$ | |
| $Ar_7$ | $CH_3$ | H | $CH_3$ | |
| $Ar_1$ | $CH_3$ | $CH_3$ | $CH_3$ | |
| $Ar_2$ | $CH_3$ | $CH_3$ | $CH_3$ | |
| $Ar_5$ | $CH_3$ | $CH_3$ | $CH_3$ | |
| $Ar_7$ | $CH_3$ | $CH_3$ | $CH_3$ | |
| $Ar_1$ | $CH_3$ | -CN | $-CH_2Cl$ | |
| $Ar_3$ | $CH_3$ | -CN | $-CH_2Cl$ | |
| $Ar_6$ | $CH_3$ | -CN | $-CH_2Cl$ | |

| Ar | $R_9$ | $R_{10}$ | $R_{14}$ | Fp. |
|---|---|---|---|---|
| $Ar_7$ | $CH_3$ | -CN | $-CH_2Cl$ | |
| $Ar_2$ | $-COCH_3$ | H | $CH_3$ | |
| $Ar_4$ | $-COCH_3$ | H | $CH_3$ | |
| $Ar_5$ | $-COCH_3$ | H | $CH_3$ | |
| $Ar_7$ | $-COCH_3$ | H | $CH_3$ | |
| $Ar_1$ | $C_6H_5$ | H | H | |
| $Ar_5$ | $C_6H_5$ | H | H | |
| $Ar_7$ | $C_6H_5$ | H | H | |
| $Ar_2$ | $CH_3$ | H | $C_6H_5$ | |
| $Ar_3$ | $CH_3$ | H | $C_6H_5$ | |
| $Ar_6$ | $CH_3$ | H | $C_6H_5$ | |
| $Ar_1$ | $C_6H_5$ | H | $C_6H_5$ | |
| $Ar_4$ | $C_6H_5$ | H | $C_6H_5$ | |
| $Ar_7$ | $C_6H_5$ | H | $C_6H_5$ | |
| $Ar_1$ | $CH_3$ | H | $-NHCOCH_3$ | |
| $Ar_2$ | $CH_3$ | H | $-NHCOCH_3$ | |
| $Ar_4$ | $CH_3$ | H | $-NHCOCH_3$ | |
| $Ar_7$ | $CH_3$ | H | $-NHCOCH_3$ | |
| $Ar_1$ | $C_6H_5$ | H | $-NHCOCH_3$ | |
| $Ar_5$ | $C_6H_5$ | H | $-NHCOCH_3$ | |
| $Ar_7$ | $C_6H_5$ | H | $-NHCOCH_3$ | |
| $Ar_1$ | H | $-(CH_2)_4-$ | | |
| $Ar_2$ | H | $-(CH_2)_4-$ | | |
| $Ar_5$ | H | $-(CH_2)_4-$ | | |
| $Ar_7$ | H | $-(CH_2)_4-$ | | |
| $Ar_1$ | H | $-CO-(CH_2)_3-$ | | |
| $Ar_2$ | H | $-CO-(CH_2)_3-$ | | |
| $Ar_5$ | H | $-CO-(CH_2)_3-$ | | |
| $Ar_7$ | H | $-CO-(CH_2)_3-$ | | |
| $Ar_4$ | H | $-CO-(CH_2)_3-$ | | |

| Ar | $R_9$ | $R_{10}$ | $R_{14}$ | Fp. |
|---|---|---|---|---|
| $Ar_1$ | H | -O-Ac | H | |
| $Ar_2$ | H | -O-Ac | H | |
| $Ar_3$ | H | -O-Ac | H | |
| $Ar_4$ | H | -O-Ac | H | |
| $Ar_5$ | H | -O-Ac | H | |
| $Ar_6$ | H | -O-Ac | H | |
| $Ar_7$ | H | -O-Ac | H | |
| $Ar_1$ | H | -O-Ac | $CH_3$ | |
| $Ar_2$ | H | -O-Ac | $CH_3$ | |
| $Ar_3$ | H | -O-Ac | $CH_3$ | |
| $Ar_4$ | H | -O-Ac | $CH_3$ | |
| $Ar_5$ | H | -O-Ac | $CH_3$ | |
| $Ar_6$ | H | -O-Ac | $CH_3$ | |
| $Ar_7$ | H | -O-Ac | $CH_3$ | |

<u>1-Aryl-pyrazoline der Formel (40)</u>

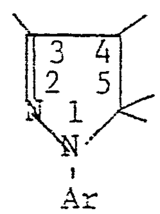

Herstellung nach Weissberger und Houben-Weyl <u>10</u>/2.

| Ar | Pos. 3 | Pos. 4 | Pos. 5 | Fp. |
|---|---|---|---|---|
| $Ar_1$ | H | H | H,H | |
| $Ar_2$ | H | H | H,H | |
| $Ar_3$ | H | H | H,H | |
| $Ar_4$ | H | H | H,H | |
| $Ar_5$ | H | H | H,H | |
| $Ar_6$ | H | H | H,H | |
| $Ar_7$ | H | H | H,H | |
| $Ar_1$ | $CH_3$ | H | H,H | |
| $Ar_2$ | $CH_3$ | H | H,H | |
| $Ar_5$ | $CH_3$ | H | H,H | |
| $Ar_7$ | $CH_3$ | H | H,H | |
| $Ar_1$ | $-NHCOCH_3$ | H | H,H | |
| $Ar_2$ | $-NHCOCH_3$ | H | H,H | |
| $Ar_3$ | $-NHCOCH_3$ | H | H,H | |
| $Ar_5$ | $-NHCOCH_3$ | H | H,H | |
| $Ar_1$ | $-NHCOCH_3$ | $CH_3$ | H,H | |
| $Ar_2$ | $-NHCOCH_3$ | $CH_3$ | H,H | |
| $Ar_4$ | $-NHCOCH_3$ | $CH_3$ | H,H | |
| $Ar_7$ | $-NHCOCH_3$ | $CH_3$ | H,H | |
| $Ar_1$ | H | H | $CH_3$, H | |
| $Ar_2$ | H | H | $CH_3$, H | |
| $Ar_3$ | H | H | $CH_3$, H | |
| $Ar_4$ | H | H | $CH_3$, H | |
| $Ar_5$ | H | H | $CH_3$, H | |
| $Ar_6$ | H | H | $CH_3$, H | |
| $Ar_7$ | H | H | $CH_3$, H | |
| $Ar_1$ | $CH_3$ | H | $CH_3$, $CH_3$ | |
| $Ar_2$ | $CH_3$ | H | $CH_3$, $CH_3$ | |
| $Ar_3$ | $CH_3$ | H | $CH_3$, $CH_3$ | |
| $Ar_4$ | $CH_3$ | H | $CH_3$, $CH_3$ | |

| Ar | Pos. 3 | Pos. 4 | Pos. 5 | Fp. |
|---|---|---|---|---|
| $Ar_5$ | $CH_3$ | H | $CH_3$, $CH_3$ | |
| $Ar_6$ | $CH_3$ | H | $CH_3$, $CH_3$ | |
| $Ar_7$ | $CH_3$ | H | $CH_3$, $CH_3$ | |
| $Ar_1$ | $C_6H_5$ | H | H, H | |
| $Ar_5$ | $C_6H_5$ | H | H,H | |
| $Ar_7$ | $C_6H_5$ | H | H,H | |
| $Ar_1$ | H | H | $C_6H_5$, H | |
| $Ar_6$ | H | H | $C_6H_5$, H | |
| $Ar_7$ | H | H | $C_6H_5$, H | |

<u>1-Aryl-2-pyrazolin-5-one der Formel (41)</u>

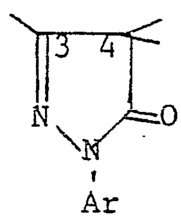

Herstellung gemäss Weissberger und Houben-Weyl <u>10</u>/2.

| Ar | Pos. 3 | Pos. 4 | Fp. |
|---|---|---|---|
| $Ar_1$ | $CH_3$ | H,H | |
| $Ar_2$ | $CH_3$ | H,H | |
| $Ar_5$ | $CH_3$ | H,H | |
| $Ar_7$ | $CH_3$ | H,H | |
| $Ar_1$ | $C_2H_5$ | H,H | |
| $Ar_3$ | $C_2H_5$ | H,H | |

| Ar | Pos. 3 | Pos. 4 | Fp. |
|---|---|---|---|
| $Ar_6$ | $C_2H_5$ | H,H | |
| $Ar_7$ | $C_2H_5$ | H,H | |
| $Ar_1$ | $C_6H_5$ | H,H | |
| $Ar_2$ | $C_6H_5$ | H,H | |
| $Ar_5$ | $C_6H_5$ | H,H | |
| $Ar_7$ | $C_6H_5$ | H,H | |
| $Ar_1$ | $-CH_2-O-C_2H_5$ | H,H | |
| $Ar_5$ | $-CH_2-O-C_2H_5$ | H,H | |
| $Ar_1$ | H | $-COOC_2H_5$, H | |
| $Ar_2$ | H | $-COOC_2H_5$,H | |
| $Ar_5$ | H | $-COOC_2H_5$, H | |
| $Ar_1$ | $CH_3$ | $CH_3$, H | |
| $Ar_2$ | $CH_3$ | $CH_3$, H | |
| $Ar_6$ | $CH_3$ | $CH_3$, H | |
| $Ar_1$ | $C_6H_5$ | $CH_3$, H | |
| $Ar_5$ | $C_6H_5$ | $CH_3$, H | |
| $Ar_7$ | $C_6H_5$ | $CH_3$, H | |
| $Ar_1$ | H | $C_6H_5$, H | |
| $Ar_5$ | H | $C_6H_5$, H | |
| $Ar_7$ | H | $C_6H_5$, H | |
| $Ar_1$ | $-(CH_2-)_4-$ | | |
| $Ar_2$ | $-(CH_2)_4-$ | | |
| $Ar_3$ | $-(CH_2)_4-$ | | |
| $Ar_4$ | $-(CH_2)_4-$ | | |
| $Ar_5$ | $-(CH_2)_4-$ | | |
| $Ar_6$ | $-(CH_2)_4-$ | | |
| $Ar_7$ | $-(CH_2)_4-$ | | |

1-Aryl-3-pyrazolin-5-one der Formel (42)

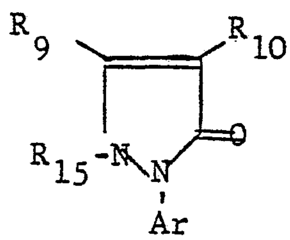

Herstellung nach Weissberger und Houben-Weyl 10/2

| Ar | $R_{15}$ | $R_9$ | $R_{10}$ | Fp. |
|---|---|---|---|---|
| $Ar_1$ | $CH_3$ | $CH_3$ | H | |
| $Ar_2$ | $CH_3$ | $CH_3$ | H | |
| $Ar_3$ | $CH_3$ | $CH_3$ | H | |
| $Ar_4$ | $CH_3$ | $CH_3$ | H | |
| $Ar_5$ | $CH_3$ | $CH_3$ | H | |
| $Ar_6$ | $CH_3$ | $CH_3$ | H | |
| $Ar_7$ | $CH_3$ | $CH_3$ | H | |
| $Ar_1$ | $n-C_4H_9$ | $CH_3$ | H | |
| $Ar_2$ | $n-C_4H_9$ | $CH_3$ | H | |
| $Ar_5$ | $n-C_4H_9$ | $CH_3$ | H | |
| $Ar_7$ | $n-C_4H_9$ | $CH_3$ | H | |
| $Ar_1$ | $CH_3$ | H | H | |
| $Ar_2$ | $CH_3$ | H | H | |
| $Ar_5$ | $CH_3$ | H | H | |

0011693



1-Aryl-pyrazolidin-3-one der Formel (43)

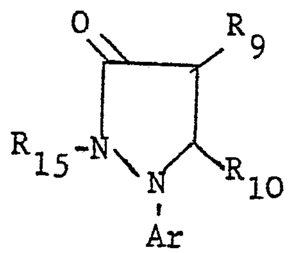

Herstellung gemäss Weissberger

| Ar | $R_{15}$ | $R_9$ | $R_{10}$ | Fp. |
|---|---|---|---|---|
| $Ar_1$ | $-COCH_3$ | H | H | |
| $Ar_2$ | $-COCH_3$ | H | H | |
| $Ar_3$ | $-COCH_3$ | H | H | |
| $Ar_4$ | $-COCH_3$ | H | H | |
| $Ar_5$ | $-COCH_3$ | H | H | |
| $Ar_6$ | $-COCH_3$ | H | H | |
| $Ar_7$ | $-COCH_3$ | H | H | |

1-Aryl-pyrazolidin-5-one der Formel (44)

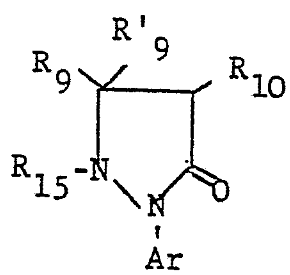

Herstellung analog zu Weissberger

| Ar | $R_{15}$ | $R_9$ | $R'_9$ | $R_{10}$ | Fp. |
|---|---|---|---|---|---|
| $Ar_1$ | $CH_3$ | H | H | H | |
| $Ar_2$ | $CH_3$ | H | H | H | |
| $Ar_3$ | $CH_3$ | H | H | H | |
| $Ar_4$ | $CH_3$ | H | H | H | |
| $Ar_5$ | $CH_3$ | H | H | H | |
| $Ar_6$ | $n-C_4H_9$ | H | H | H | |
| $Ar_7$ | $n-C_4H_9$ | H | H | H | |
| $Ar_1$ | $-COCH_3$ | H | H | H | |
| $Ar_2$ | $-COCH_3$ | H | H | H | |
| $Ar_5$ | $-COCH_3$ | H | H | H | |
| $Ar_7$ | $-COCH_3$ | H | H | H | |
| $Ar_1$ | $CH_3$ | H | H | $CH_3$ | |
| $Ar_2$ | $CH_3$ | H | H | $CH_3$ | |
| $Ar_4$ | $CH_3$ | H | H | $CH_3$ | |
| $Ar_5$ | $CH_3$ | H | H | $CH_3$ | |
| $Ar_1$ | $CH_3$ | H | H | $C_2H_5$ | |
| $Ar_5$ | $CH_3$ | H | H | $C_2H_5$ | |
| $Ar_7$ | $CH_3$ | H | H | $C_2H_5$ | |
| $Ar_1$ | $CH_3$ | $CH_3$ | $CH_3$ | H | |
| $Ar_5$ | $CH_3$ | $CH_3$ | $CH_3$ | H | |
| $Ar_7$ | $CH_3$ | $CH_3$ | $CH_3$ | H | |

1-Aryl-pyrazolin-3-one der Formel(45)

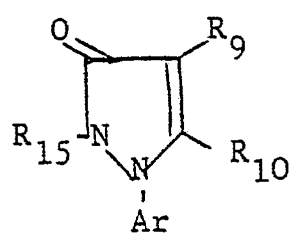

Herstellung nach Weissberger

| Ar | $R_{15}$ | $R_9$ | $R_{10}$ | Fp. |
|---|---|---|---|---|
| $Ar_1$ | $CH_3$ | H | H | |
| $Ar_2$ | $CH_3$ | H | H | |
| $Ar_3$ | $CH_3$ | H | H | |
| $Ar_4$ | $CH_3$ | H | H | |
| $Ar_5$ | $CH_3$ | H | H | |
| $Ar_6$ | $CH_3$ | H | H | |
| $Ar_7$ | $CH_3$ | H | H | |
| $Ar_1$ | $CH_3$ | H | $CH_3$ | |
| $Ar_2$ | $CH_3$ | H | $CH_3$ | |
| $Ar_4$ | $CH_3$ | H | $CH_3$ | |
| $Ar_5$ | $CH_3$ | H | $CH_3$ | |
| $Ar_7$ | $CH_3$ | H | $CH_3$ | |

<u>1-Aryl-pyrazolin-4-one der Formel (46)</u>

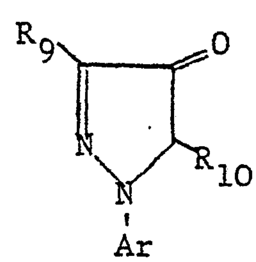

Herstellung gemäss Weissberger

| Ar | $R_9$ | $R_{10}$ | Fp. |
|---|---|---|---|
| $Ar_1$ | H | H | |
| $Ar_2$ | H | H | |
| $Ar_4$ | H | H | |
| $Ar_5$ | H | H | |
| $Ar_7$ | H | H | |
| $Ar_1$ | H | $CH_3$ | |
| $Ar_2$ | H | $CH_3$ | |
| $Ar_3$ | H | $CH_3$ | |
| $Ar_4$ | H | $CH_3$ | |
| $Ar_5$ | H | $CH_3$ | |
| $Ar_6$ | H | $CH_3$ | |
| $Ar_7$ | H | $CH_3$ | |

<u>1-Aryl-pyrazolidin-3,5-dione der Formel (47)</u>

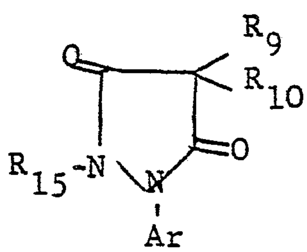

Herstellung nach Weissberger

| Ar | $R_{15}$ | $R_9$ | $R_{10}$ | Fp. |
|---|---|---|---|---|
| $Ar_1$ | $CH_3$ | H | H | |
| $Ar_2$ | $CH_3$ | H | H | |
| $Ar_5$ | $CH_3$ | H | H | |
| $Ar_7$ | $CH_3$ | H | H | |

| Ar | $R_{15}$ | $R_9$ | $R_{10}$ | Fp. |
|---|---|---|---|---|
| $Ar_1$ | $CH_3$ | H | $CH_3$ | |
| $Ar_2$ | $CH_3$ | H | $CH_3$ | |
| $Ar_4$ | $CH_3$ | H | $CH_3$ | |
| $Ar_5$ | $CH_3$ | H | $CH_3$ | |
| $Ar_7$ | $CH_3$ | H | $CH_3$ | |
| $Ar_1$ | $CH_3$ | $CH_3$ | $CH_3$ | |
| $Ar_2$ | $CH_3$ | $CH_3$ | $CH_3$ | |
| $Ar_3$ | $CH_3$ | $CH_3$ | $CH_3$ | |
| $Ar_6$ | $CH_3$ | $CH_3$ | $CH_3$ | |
| $Ar_7$ | $CH_3$ | $CH_3$ | $CH_3$ | |
| $Ar_1$ | $CH_3$ | H | Benzyl | |
| $Ar_2$ | $CH_3$ | H | Benzyl | |
| $Ar_4$ | $CH_3$ | H | Benzyl | |
| $Ar_5$ | $CH_3$ | H | Benzyl | |
| $Ar_7$ | $CH_3$ | H | Benzyl | |
| $Ar_1$ | $CH_3$ | H | $C_6H_5$ | |
| $Ar_2$ | $CH_3$ | H | $C_6H_5$ | |
| $Ar_3$ | $CH_3$ | H | $C_6H_5$ | |
| $Ar_4$ | $CH_3$ | H | $C_6H_5$ | |
| $Ar_5$ | $CH_3$ | H | $C_6H_5$ | |
| $Ar_6$ | $CH_3$ | H | $C_6H_5$ | |
| $Ar_7$ | $CH_3$ | H | $C_6H_5$ | |

Sultame der Formel (48)

Herstellung in Analogie zu: Org.Prep.Proced.Int. 9 (1), 27-33 (1978)
Annalen der Chemie: 1974, S. 667
Annalen der Chemie: 761, 115 (1972)
Annalen der Chemie 647, 37 (1961)

A) Fünfring-Sultame der Formel

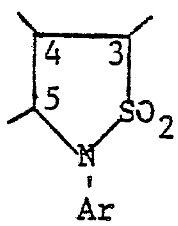

(48a)

| Ar | Pos. 3 | Pos. 4 | Pos. 5 | Fp. |
|---|---|---|---|---|
| $Ar_1$ | H | H | H | 109-113° |
| $Ar_2$ | H | H | H | 150-151° |
| $Ar_3$ | H | H | H | |
| $Ar_4$ | H | H | H | 144° |
| $Ar_5$ | H | H | H | |
| $Ar_6$ | H | H | H | 143° |
| $Ar_7$ | H | H | H | 122-124° |
| $Ar_8$ | H | H | H | 131-132° |
| $Ar_9$ | H | H | H | |
| $Ar_1$ | H | $CH_3$ | H | |
| $Ar_2$ | H | $CH_3$ | H | |

| Ar | Pos.3 | Pos. 4 | Pos. 5 | Fp. |
|---|---|---|---|---|
| $Ar_1$ | H | H | $CH_3$ | |
| $Ar_2$ | H | H | $CH_3$ | |
| $Ar_5$ | H | H | $CH_3$ | |
| $Ar_7$ | H | H | $CH_3$ | |
| $Ar_1$ | $CH_3$ | H | H | |
| $Ar_2$ | $CH_3$ | H | H | |
| $Ar_4$ | $CH_3$ | H | H | |
| $Ar_7$ | $CH_3$ | H | H | |

Sultame der Formel

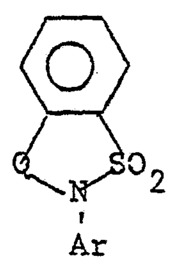

(48b)

| Ar | Q | Fp. |
|---|---|---|
| $Ar_1$ | $-CH_2-$ | |
| $Ar_2$ | $-CH_2-$ | 97° |
| $Ar_3$ | $-CH_2-$ | |
| $Ar_4$ | $-CH_2-$ | |
| $Ar_5$ | $-CH_2-$ | |
| $Ar_6$ | $-CH_2-$ | |
| $Ar_7$ | $-CH_2-$ | |
| $Ar_8$ | $-CH_2-$ | 212° |
| $Ar_1$ | -CO- | |
| $Ar_2$ | -CO- | |
| $Ar_4$ | -CO- | |
| $Ar_5$ | -CO- | |
| Ar7 | -CO- | |

B.) Sechsring-Sultame der Formel (49)

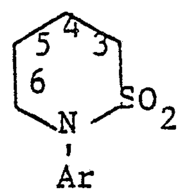

| Ar | Pos. 3 | Pos. 4 | Pos. 5 | Pos. 6 | Fp. |
|---|---|---|---|---|---|
| $Ar_1$ | H | H | H | H | |
| $Ar_2$ | H | H | H | H | |
| $Ar_3$ | H | H | H | H | |
| $Ar_4$ | H | H | H | H | |
| $Ar_5$ | H | H | H | H | |
| $Ar_6$ | H | H | H | H | |
| $Ar_7$ | H | H | H | H | |
| $Ar_9$ | H | H | H | H | |
| $Ar_1$ | $CH_3$ | H | H | H | |
| $Ar_2$ | $CH_3$ | H | H | H | |
| $Ar_4$ | $CH_3$ | H | H | H | |
| $Ar_5$ | $CH_3$ | H | H | H | |
| $Ar_1$ | H | H | H | $CH_3$ | |
| $Ar_2$ | H | H | H | $CH_3$ | |
| $Ar_4$ | H | H | H | $CH_3$ | |
| $Ar_5$ | H | H | H | $CH_3$ | |

0011693



Sultame der Formel

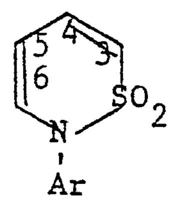

| Ar | Pos. 3 | Pos. 4 | Pos. 5 | Pos. 6 |
|---|---|---|---|---|
| $Ar_1$ | H | $CH_3$ | H | $CH_3$ |
| $Ar_2$ | H | $CH_3$ | H | $CH_3$ |
| $Ar_3$ | H | $CH_3$ | H | $CH_3$ |
| $Ar_4$ | H | $CH_3$ | H | $CH_3$ |
| $Ar_5$ | H | $CH_3$ | H | $CH_3$ |
| $Ar_6$ | H | $CH_3$ | H | $CH_3$ |
| $Ar_7$ | H | $CH_3$ | H | $CH_3$ |
| $Ar_8$ | H | $CH_3$ | H | $CH_3$ |

<u>4-Aryl-urazole der Formel (50)</u>

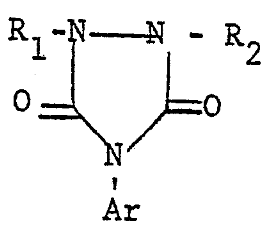

Herstellung in Analogie zu USP 3579500

| Ar | $R_1$ | $R_2$ | Fp. |
|---|---|---|---|
| $Ar_1$ | H | H | |
| $Ar_2$ | H | H | |
| $Ar_2$ | $CH_3$ | $CH_3$ | ca.260° |
| $Ar_4$ | $CH_3$ | $CH_3$ | |
| $Ar_5$ | $CH_3$ | H | |
| $Ar_1$ | $C_3H_7$ | $C_3H_7$ | |
| $Ar_7$ | $CH_3$ | $C_2H_5$ | |
| $Ar_2$ | $-COCH_3$ | $-COCH_3$ | |
| $Ar_3$ | $-COCH_3$ | $-COCH_3$ | |
| $Ar_1$ | $-CO-NH-CH_3$ | H | |
| $Ar_2$ | $-CO-NH-CH_3$ | $-CONHCH_3$ | |
| $Ar_3$ | $CH_3$ | $-CO-CH_3$ | |
| $Ar_2$ | $-COOC_2H_5$ | $-COOC_2H_5$ | |
| $Ar_2$ | $CH_3$ | $-CONHCH_3$ | |
| $Ar_3$ | $CH_3$ | $C_6H_5$ | |
| $Ar_1$ | $-(CH_2)_4-$ | | |
| $Ar_7$ | $n-C_4H_9$ | $n-C_4H_9$ | |
| $Ar_3$ | $i-C_4H_9$ | $i-C_4H_9$ | |
| $Ar_5$ | $-CONHC_2H_5$ | $-CO-NHC_2H_5$ | |
| $Ar_1$ | $-COOCH_3$ | $-CONHCH_3$ | |

4-Aryl-1,2,4-triazolidin-3-one der Formel (51)

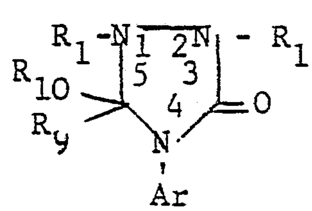

Herstellung analog zu USP 3 922 162

| Ar | $R_1$ Pos. 1 | $R_1$ Pos. 2 | $R_9$ | $R_{10}$ | Fp. |
|---|---|---|---|---|---|
| $Ar_1$ | H | H | H | H | |
| $Ar_2$ | H | H | H | H | |
| $Ar_1$ | H | $CH_3$ | H | H | |
| $Ar_2$ | H | $CH_3$ | H | H | |
| $Ar_3$ | H | $CH_3$ | H | H | |
| $Ar_4$ | H | $CH_3$ | H | H | |
| $Ar_5$ | H | $i\text{-}C_3H_7$ | H | H | |
| $Ar_6$ | H | $i\text{-}C_3H_7$ | H | H | |
| $Ar_2$ | $CH_3$ | $CH_3$ | H | H | |
| $Ar_1$ | $CH_3$ | $C_4H_9$ | H | H | |
| $Ar_7$ | H | $CH_3$ | $CH_3$ | H | |
| $Ar_2$ | H | $CH_3$ | $CH_3$ | H | |
| $Ar_2$ | H | $C_2H_5$ | $-(CH_2)_4-$ | | |
| $Ar_3$ | $-COCH_3$ | $CH_3$ | H | H | |
| $Ar_1$ | $C_4H_9$ | $C_2H_5$ | H | H | |

<u>4-Aryl-1,2,4-triazole der Formel (52)</u>

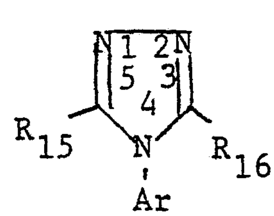

Herstellung analog Weissberger

| Ar | $R_{15}$ | $R_{16}$ | Fp. |
|---|---|---|---|
| $Ar_1$ | -OAc | -OAc | |
| $Ar_2$ | -O-Ac | -OAc | |
| $Ar_5$ | -O-Ac | -OAc | |
| $Ar_1$ | $-SCH_3$ | $-O-COCH_3$ | |
| $Ar_2$ | $-SCH_3$ | $-O-COCH_3$ | |
| $Ar_5$ | $-S-C_4H_9(n)$ | $-O-COCH_3$ | |
| $Ar_7$ | $-SC_4H_9(n)$ | $-O-COCH_3$ | |
| $Ar_1$ | $-SCH_3$ | $-O-CON(CH_3)_2$ | |
| $Ar_2$ | $-SCH_3$ | $-O-CON(CH_3)_2$ | |
| $Ar_4$ | $-SCH_3$ | $-O-CON(CH_3)_2$ | |
| $Ar_7$ | $-SCH_3$ | $-O-CON(CH_3)_2$ | |
| $Ar_1$ | $-SCH_2-CH=CH_2$ | $-O-CON(CH_3)_2$ | |
| $Ar_2$ | $-SCH_2-CH=CH_2$ | $-O-CON(CH_3)_2$ | |
| $Ar_5$ | $-SCH_2-CH=CH_2$ | $-O-CON(CH_3)_2$ | |
| $Ar_7$ | $-S-CH_2-CH=CH_2$ | $-O-CON(CH_3)_2$ | |

4-Aryl-1,2,4-triazolin-3-one der Formel (53)

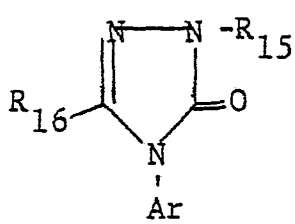

| Ar | $R_{15}$ | $R_{16}$ | Fp. |
|---|---|---|---|
| $Ar_1$ | $CH_3$ | $-SCH_3$ | |
| $Ar_2$ | $CH_3$ | $-SCH_3$ | |
| $Ar_5$ | $CH_3$ | $-SCH_3$ | |
| $Ar_1$ | $C_2H_5$ | $-SC_2H_5$ | |
| $Ar_4$ | $C_2H_5$ | $-SC_2H_5$ | |
| $Ar_6$ | $C_2H_5$ | $-SC_2H_5$ | |
| $Ar_1$ | $n-C_4H_9$ | $-S-C_4H_9(n)$ | |
| $Ar_3$ | $n-C_4H_9$ | $-S-C_4H_9(n)$ | |
| $Ar_7$ | $n-C_4H_9$ | $-S-C_4H_9(n)$ | |

Herstellung analog zu USP 3 922 162

1-Aryl-1,2,4-triazole der Formel (54)

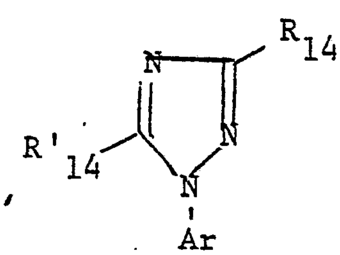

Herstellung nach Houben-Weyl und USP 3 489 761 und 4 096 035

| Ar | $R_{14}$ | $R'_{14}$ | Fp. |
|---|---|---|---|
| $Ar_1$ | $-COOC_2H_5$ | $-O-COCH_3$ | |
| $Ar_2$ | $-COOC_2H_5$ | $-O-COCH_3$ | |
| $Ar_5$ | $-COOC_2H_5$ | $-O-COCH_3$ | |
| $Ar_7$ | $-COOC_2H_5$ | $-O-COCH_3$ | |

sowie die Verbindungen, in denen $R_{14}$ und $R'_{14}$ Wasserstoff bedeuten und Ar der 2-Trifluormethansulfonamido-4-chlor-phenyl-(1)-Rest bezw. der 2-Trifluormethyl-4-trifluormethansulfonamido-phenyl(1)Rest ist.

1-Aryl-1,2,3-triazole der Formel (55)

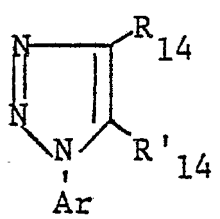

Herstellung analog französischer Patentschrift 2 268 018

| Ar | $R_{14}$ | $R'_{14}$ | Fp. |
|---|---|---|---|
| $Ar_1$ | H | H | |
| $Ar_1$ | $-COOCH_3$ | $C_6H_5$ | |
| $Ar_2$ | $-COOCH_3$ | $C_6H_5$ | |
| $Ar_1$ | H | $C_6H_5$ | |
| $Ar_2$ | H | $C_6H_5$ | |
| $Ar_2$ | H | $CH_3$ | |
| $Ar_2$ | $-COOC_2H_5$ | $CH_3$ | |
| $Ar_3$ | $-COOCH_2CH_2-OCH_3$ | $C_6H_5$ | |
| $Ar_4$ | H | $C_2H_5$ | |
| $Ar_1$ | -COOH | H | |

3-Aryl-1,3,4-Oxa(und thia-)diazolin-2-one der Formel (56)

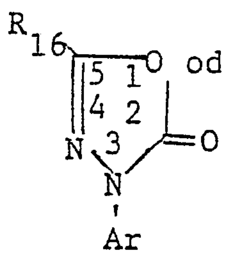

oder S

| Ar | Pos. 1 (Heteroatom) | $R_{16}$ | Fp. |
|---|---|---|---|
| $Ar_1$ | O | $-OC_2H_5$ | |
| $Ar_2$ | O | $-OC_2H_5$ | |
| $Ar_5$ | O | $-OC_2H_5$ | |
| $Ar_7$ | O | $-OC_2H_5$ | |
| $Ar_1$ | S | $-OC_2H_5$ | |
| $Ar_2$ | S | $-OC_2H_5$ | |
| $Ar_4$ | S | $-OC_2H_5$ | |
| $Ar_5$ | S | $-OC_2H_5$ | |
| $Ar_7$ | S | $-OC_2H_5$ | |

2-Aryl-1,2,3-thiadiazolin-S-dioxide der Formel (57)

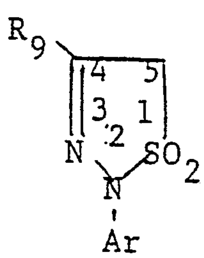

Herstellung gemäss Weissberger

| Ar | $R_9$ | Fp |
|---|---|---|
| $Ar_1$ | $CH_3$ | |
| $Ar_2$ | $CH_3$ | |
| $Ar_3$ | $CH_3$ | |
| $Ar_4$ | $CH_3$ | |
| $Ar_5$ | $CH_3$ | |
| $Ar_6$ | $CH_3$ | |
| $Ar_7$ | $CH_3$ | |
| $Ar_1$ | $C_6H_5$ | |
| $Ar_5$ | $C_6H_5$ | |
| $Ar_7$ | $C_6H_5$ | |

<u>2-Aryl-tetrahydro-1,2,4-triazin-3,5-dione der Formel (58)</u>

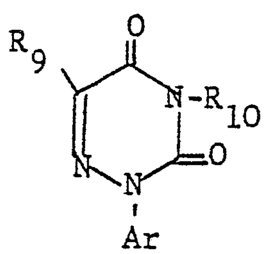

(58)

Herstellung nach bekannten Methoden gemäss dem Schema:

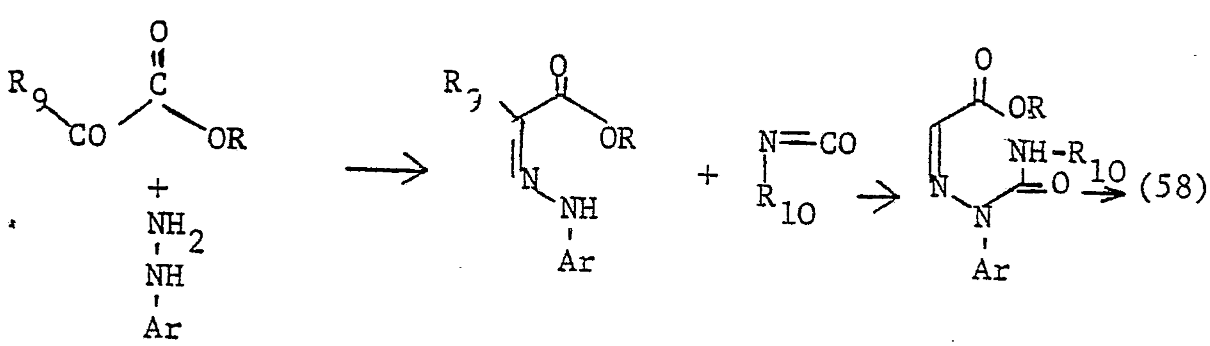

| Ar | $R_{10}$ | $R_9$ | Fp. |
|---|---|---|---|
| $Ar_1$ | $C_2H_5$ | $C_2H_5$ | |
| $Ar_1$ | $CH_3$ | $i-C_3H_7$ | |
| $Ar_1$ | $C_6H_5$ | $CH_3$ | |
| $Ar_2$ | $CH_3$ | $CH_3$ | |
| $Ar_2$ | $CH_3$ | $i-C_3H_7$ | |
| $Ar_2$ | $C_2H_5$ | $CH_3$ | |
| $Ar_2$ | $i-C_3H_7$ | $i-C_3H_7$ | |
| $Ar_3$ | $C_2H_5$ | $C_2H_5$ | |
| $Ar_3$ | $CH_3$ | $i-C_3H_7$ | |
| $Ar_3$ | $CH_3$ | $C_6H_5$ | |
| $Ar_3$ | $CH_3$ | $CH_3$ | |
| $Ar_4$ | $C_2H_5$ | $CH_3$ | |
| $Ar_4$ | $CH_3$ | $C_2H_5$ | |
| $Ar_4$ | $CH_3$ | $i-C_3H_7$ | |
| $Ar_4$ | $CH_3$ | $CH_3$ | |
| $Ar_5$ | $CH_3$ | $CH_3$ | |
| $Ar_5$ | $CH_3$ | $C_2H_5$ | |
| $Ar_5$ | $CH_3$ | $i-C_3H_7$ | |
| $Ar_5$ | $C_2H_5$ | $C_2H_5$ | |
| $Ar_6$ | $CH_3$ | $CH_3$ | |
| $Ar_6$ | $CH_3$ | $C_2H_5$ | |
| $Ar_6$ | $CH_3$ | $i-C_3H_7$ | |
| $Ar_7$ | $CH_3$ | $CH_3$ | |
| $Ar_7$ | $CH_3$ | $C_2H_5$ | |
| $Ar_7$ | $CH_3$ | $i-C_3H_7$ | |
| $Ar_7$ | $C_6H_5$ | $i-C_3H_7$ | |

1-Aryl-tetrahydro-1,3,5-triazin-2,6-dione der Formel (59)

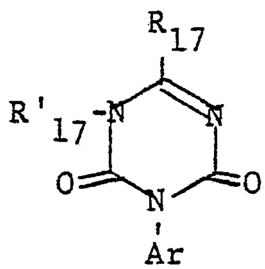

Herstellung in Analogie zu DOS 2 254 200 und 2 236 312

| $Ar_1$ | $R_{17}$ | $R'_{17}$ | Fp. |
|---|---|---|---|
| $Ar_1$ | H | $CH_3$ | |
| $Ar_2$ | H | $C_2H_5$ | |
| $Ar_3$ | H | $C_2H_5$ | |
| $Ar_4$ | H | $C_2H_5$ | |
| $Ar_5$ | H | $C_2H_5$ | |
| $Ar_6$ | $-OCH_3$ | $CH_3$ | |
| $Ar_7$ | $-OCH_3$ | $i-C_3H_7$ | |
| $Ar_2$ | $-SCH_3$ | $CH_3$ | |
| $Ar_2$ | Cl | $CH_3$ | |
| $Ar_2$ | $-N(CH_3)_2$ | $CH_3$ | |
| $Ar_4$ | $-OCH_3$ | H | |
| $Ar_1$ | $-OCOCH_3$ | $CH_3$ | |
| $Ar_1$ | $-NHC_2H_5$ | $CH_3$ | |
| $Ar_2$ | $-OC_2H_5$ | Cyclohexyl | |

1-Aryl-hexahydro-1,3,5-triazin-2-one der Formel (60)

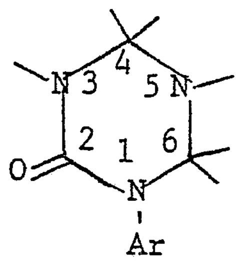

Herstellung analog zu
USP 3 698 886 und
DAS 1 249 003

| Ar | Pos. 3 | Pos. 4 | Pos. 5 | Pos. 6 | Fp. |
|---|---|---|---|---|---|
| $Ar_1$ | $CH_3$ | H,H | $CH_3$ | H,H | |
| $Ar_2$ | $CH_3$ | H,H | $CH_3$ | H,H | |
| $Ar_3$ | $CH_3$ | H,H | $CH_3$ | H,H | |
| $Ar_4$ | $CH_3$ | H,H | $CH_3$ | H,H | |
| $Ar_5$ | $C_2H_5$ | H,H | $CH_3$ | H,H | |
| $Ar_6$ | $CH_3$ | H,H | $C_2H_5$ | H,H | |
| $Ar_7$ | H | H,H | H | H,H | |
| $Ar_2$ | $CH_3$ | $i\text{-}C_3H_7$,H | $CH_3$ | $i\text{-}C_3H_7$,H | |
| $Ar_3$ | $C_2H_5$ | $n\text{-}C_4H_9$,H | $C_2H_5$ | $n\text{-}C_4H_9$,H | |
| $Ar_2$ | $CH_3$ | $C_2H_5$,H | $CH_3$ | $C_2H_5$,H | |
| $Ar_1$ | $CH_3$ | $CH_3$,H | $CH_3$ | $CH_3$,H | |

4-Aryl-dihydro-1,3,4-oxa(und thia-)diazin-5-one
der Formel (61)

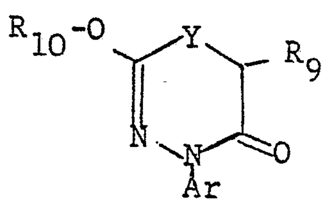

(61)

Herstellung nach dem Schema:

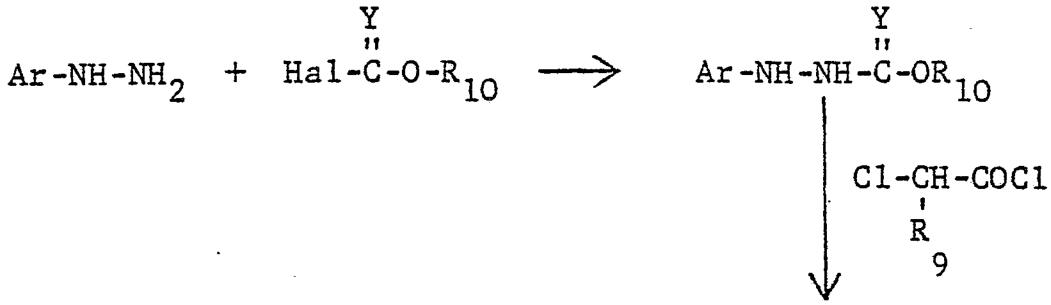

(61)

| Ar | $R_{10}$ | $R_9$ | Y | Fp. |
|---|---|---|---|---|
| $Ar_1$ | $C_2H_5$ | H | O | |
| $Ar_1$ | $C_2H_5$ | H | S | |
| $Ar_1$ | $C_2H_5$ | $CH_3$ | S | |
| $Ar_1$ | $C_2H_5$ | H | $SO_2$ | |
| $Ar_2$ | $CH_3$ | H | O | |
| $Ar_2$ | $CH_3$ | H | S | |
| $Ar_2$ | $CH_3$ | $CH_3$ | S | |
| $Ar_3$ | $CH_3$ | H | O | |
| $Ar_3$ | $CH_3$ | H | S | |
| $Ar_4$ | $C_2H_5$ | H | O | |
| $Ar_4$ | $C_2H_5$ | H | S | |
| $Ar_5$ | $C_2H_5$ | H | O | |
| $Ar_5$ | $C_2H_5$ | H | S | |
| $Ar_5$ | $C_2H_5$ | $CH_3$ | S | |
| $Ar_6$ | $CH_3$ | H | O | |
| $Ar_6$ | $CH_3$ | H | S | |
| $Ar_6$ | $CH_3$ | $CH_3$ | S | |
| $Ar_7$ | -Benzyl | H | O | |
| $Ar_7$ | -Benzyl | H | S | |
| $Ar_7$ | $C_2H_5$ | $CH_3$ | S | |
| $Ar_7$ | $C_2H_5$ | $C_6H_5$ | S | |

Die Herstellung erfindungsgemässer Mittel erfolgt in an sich bekannter Weise durch inniges Vermischen und Vermahlen von Wirkstoffen der Formel I mit geeigneten Trägerstoffen, gegebenenfalls unter Zusatz von gegenüber den Wirkstoffen inerten Dispersions- oder Lösungsmitteln. Die Wirkstoffe können in den folgenden Aufarbeitungsformen vorliegen und angewendet werden:

| | |
|---|---|
| feste Aufarbeitungsformen: | Stäubemittel, Streumittel, Granulate, Umhüllungsgranulate, Imprägnierungsgranulate und Homogengranulate; |
| in Wasser dispergierbare Wirkstoffkonzentrate: | Spritzpulver, (wettable powder), Pasten, Emulsionen; |
| flüssige Aufarbeitungsformen: | Lösungen. |

Zur Herstellung fester Aufarbeitungsformen (Stäubemittel, Streumittel, Granulate) werden die Wirkstoffe mit festen Trägerstoffen vermischt. Als Trägerstoffe kommen zum Beispiel Kaolin, Talkum, Bolus, Löss, Kreide, Kalkstein, Kalkgrits, Ataclay, Dolomit, Diatomeenerde, gefällte Kieselsäure, Erdalkalisilikate, Natrium- und Kaliumaluminiumsilikate (Feldspäte und Glimmer), Calcium- und Magnesiumsulfate, Magnesiumoxid, gemahlene Kunststoffe, Düngemittel, wie Ammoniumsulfat, Ammoniumphosphat, Ammoniumnitrat, Harnstoff, gemahlene pflanzliche Produkte, wie Getreidemehl, Baumrindemehl, Holzmehl, Nussschalenmehl, Cellulosepulver, Rückstände von Pflanzenextraktionen, Aktivkohle etc., je für sich oder als Mischungen untereinander in Frage.

Granulate lassen sich herstellen, indem man die Wirkstoffe in einem organischen Lösungsmittel löst und die so erhaltene Lösung auf ein granuliertes Mineral, z.B. Attapul $SiO_2$, Granicalcium oder Bentonit, aufbringt und dann das organische Lösungsmittel wieder verdampft.

Polymerengranulate können hergestellt werden, indem ma z.B. ein fertiges, poröses Polymerengranulat, wie Harnstoff/Formaldehyd-Polymerisate, Polyacrylnitril und Polyester, mit bestimmter Oberfläche und günstigem vorausbestimmtem Absorptions/Desorptionsverhältnis mit den Wirkstoffen, z.B. in Form ihrer Lösungen (in einem niedrig siedenden Lösungsmittel), imprägniert und das Lösungsmittel entfernt. Derartige Polymerengranulate können in Form von Mikrogranulaten mit Schüttgewichten von vorzugsweise 300 g/Liter bis 600 g/Liter auch mit Hilfe von Zerstäubern aufgebracht werden. Das Zerstäuben kann über ausgedehnte Behandlungsflächen mit Hilfe von Flugzeugen durchgeführt we:

Granulate sind auch durch Kompaktieren des Trägermate: mit den Wirk- und Zusatzstoffen und anschliessendes Zerklei: erhältlich.

Diesen Mitteln können ferner den Wirkstoff stabilisie: Zusätze und/oder nichtionische, anionaktive und kationenaktive Stoffe zugegeben werden, die beispielsweise die Haftfestigkeit der Wirkstoffe auf Pflanzen und Pflanzenteil verbessern (Haft- und Klebemittel) und/oder eine bessere Benetzbarkeit (Netzmittel) sowie Dispergierbarkeit (Dispergatoren) gewährleisten. Als Klebemittel kommen beispielsweis die folgenden in Frage: Olein-Kalk-Mischung, Cellulosederiva (Methylcellulose, Carboxymethylcellulose), Hydroxyäthylenglykoläther von Mono- und Dialkylphenolen mit 5 bis 15 Aethylenoxidresten pro Molekül und 8 bis 9 Kohlenstoffatomer im Alkylrest, Ligninsulfonsäure, deren Alkalimetall- und

Erdalkalimetallsalze, Polyäthylenglykoläther (Carbowaxe), Fettalkoholpolyglykoläther mit 5 bis 20 Aethylenoxidresten pro Molekül und 8 bis 18 Kohlenstoffatomen im Fettalkoholteil, Kondensationsprodukte von Aethylenoxid, Propylenoxid, Polyvinylpyrrolidone, Polyvinylalkohole, Kondensationsprodukte von Harnstoff-Formaldehyd sowie Latex-Produkte.

In Wasser dispergierbare Wirkstoffkonzentrate, d.h. Spritzpulver (wettable powder), Pasten und Emulsionskonzentrate stellen Mittel dar, die mit Wasser auf jede gewünschte Konzentration verdünnt werden können. Sie bestehen aus Wirkstoff, Trägerstoff, gegebenenfalls den Wirkstoff stabilisierenden Zusätzen, oberflächenaktiven Substanzen und Antischaummitteln und gegebenenfalls Lösungsmitteln.

Die Spritzpulver (wettable powder) und Pasten werden erhalten, indem man die Wirkstoffe mit Dispergiermitteln und pulverförmigen Trägerstoffen in geeigneten Vorrichtungen bis zur Homogenität vermischt und vermahlt. Als Trägerstoffe kommen beispielsweise die vorstehend für die festen Aufarbeitungsformen erwähnten in Frage. In manchen Fällen ist es vorteilhaft, Mischungen verschiedener Trägerstoffe zu verwenden. Als Dispergatoren können beispielsweise verwendet werden: Kondensationsprodukte von sulfoniertem Naphthalin und sulfonierten Naphthalinderivaten mit Formaldehyd, Kondensationsprodukte des Naphthalins bzw. von Naphthalinsulfonsäuren mit Phenol und Formaldehyd sowie Alkalimetall-, Ammonium- und Erdalkalimetallsalze von Ligningsulfonsäure, weiter Alkylarylsulfonate, Alkali- und Erdalkalimetallsalze der Dibutylnaphthalinsulfonsäure, Fettalkoholsulfate, wie Salze sulfatierter Hexadecanole, Heptadecanole und Salze von sulfatiertem Fettalkoholpolyäthylenglykoläther, das

Natriumsalz von Oleylmethyltaurid, ditertiäre Acetylenglykole, Dialkyldilaurylammoniumchlorid und fettsaure Alkali und Erdalkalimetallsalze.

Als Antischaummittel kommen zum Beispiel Silicone in Frage.

Die Wirkstoffe werden mit den oben aufgeführten Zusätz so vermischt, vermahlen, gesiebt und passiert, dass bei den Spritzpulvern der feste Anteil eine Korngrösse von 0,02 bis 0,04 und bei den Pasten von 0,03 mm nicht überschreitet. Zur Herstellung von Emulsionskonzentraten und Pasten werden Dispergiermittel, wie sie in den vorangehenden Abschnitten aufgeführt wurden, organische Lösungsmittel und Wasser verwendet. Als Lösungsmittel kommen beispielsweise die folgenden in Frage: Alkohole, Benzol, Xylole, Toluol, Dimethylsulfoxid, N,N-dialkylierte Amide und Trialkylamine. Die Lösungsmittel müssen praktisch geruchlos, nicht phytotoxisch, den Wirkstoffen gegenüber inert und dürfen nicht leicht brennbar sein.

Ferner können die erfindungsgemässen Mittel in Form vc Lösungen angewendet werden. Hierzu wird der Wirkstoff bzw. werden mehrere Wirkstoffe der Formel I in geeigneten organis Lösungsmitteln, Lösungsmittelgemischen, Wasser oder Gemische von organischen Lösungsmitteln mit Wasser gelöst. Als organ: Lösungsmittel können aliphatische und aromatische Kohlenwasserstoffe, deren chlorierte Derivate, Alkylnaphthaline, allein oder als Mischung untereinander verwendet werden.

Der Gehalt an Wirkstoff in den oben beschriebenen Mitteln liegt zwischen 0,1 bis 95%, bevorzugt zwischen 1 bi: 80%. Anwendungsformen können bis hinab zu 0,001% verdünnt

werden. Die Aufwandmengen betragen in der Regel 0,1 bis 10 kg AS/ha, vorzugsweise 0,25 bis 5 kg AS/ha. Die Wirkstoffe der Formel I können beispielsweise wie folgt formuliert werden (Teile bedeuten Gewichtsteile):

Stäubemittel:

Zur Herstellung eines a) 5%igen und b) 2%igen Stäubemittels werden die folgenden Stoffe verwendet:

| | | | |
|---|---|---|---|
| a) | 5 | Teile | Wirkstoff des Beispiels 1 |
| | 95 | Teile | Talkum, |
| b) | 2 | Teile | Wirkstoff des Beispiels 2 |
| | 1 | Teil | hochdisperse Kieselsäure, |
| | 97 | Teile | Talkum; |

Die Wirkstoffe werden mit den Trägerstoffen vermischt und vermahlen.

Granulat

Zur Herstellung eines 5%igen Granulates werden die folgenden Stoffe verwendet:

| | | |
|---|---|---|
| 5 | Teile | eines Wirkstoffs der allgemeinen Formel I |
| 0,25 | Teile | Epichlorhydrin |
| 0,25 | Teile | Cetylpolyäthylenglykoläther mit 8 Mol Aethylenoxid, |
| 3,50 | Teile | Polyäthylenglykol, |
| 91 | Teile | Kaolin (Korngrösse 0,3 bis 0,8 mm) |

Die Aktivsubstanz wird mit Epichlorhydrin vermischt und in 6 Teilen Aceton gelöst, hierauf werden Polyäthylenglykol und Cetylpolyäthylenglykoläther zugesetzt. Die so erhaltene Lösung wird auf Kaolin aufgesprüht und anschliessend im Vakuum verdampft.

Spritzpulver

Zur Herstellung eines a) 50%igen, b) 25%igen und c) 10%igen Spritzpulvers werden folgende Bestandteile verwendet:

| | | | |
|---|---|---|---|
| a) | 50 | Teile | Wirkstoff des Beispiels 4, |
| | 5 | Teile | Natriumdibutylnaphthylsulfonat, |
| | 3 | Teile | Naphthalinsulfonsäuren-Phenolsulfonsäuren-Formaldehyd-Kondensat 3:2:1, |
| | 20 | Teile | Kaolin, |
| | 22 | Teile | Champagne-Kreide; |
| b) | 25 | Teile | des obengenannten oder eines andern Wirkstoffs der Formel I, |
| | 5 | Teile | Oleylmethyltaurid-Natrium-Salz, |
| | 2,5 | Teile | Naphthalinsulfonsäuren-Formaldehyd-Kondensat, |
| | 0,5 | Teile | Carboxymethylcellulose, |
| | 5 | Teile | neutrales Kalium-Aluminium-Silikat, |
| | 62 | Teile | Kaolin; |
| c) | 10 | Teile | des Wirkstoffs gemäss Beispiel 3, |
| | 3 | Teile | Gemisch der Natriumsalze von gesättigten Fettalkoholsulfaten, |
| | 5 | Teile | Naphthalinsulfonsäuren-Formaldehyd-Kondensat, |
| | 82 | Teile | Kaolin. |

Der angegebene Wirkstoff wird auf die entsprechenden Trägerstoffe (Kaolin und Kreide) aufgezogen und anschliessend vermischt und vermahlen. Man erhält Spritzpulver von vorzüglicher Benetzbarkeit und Schwebefähigkeit. Aus solchen Spritzpulvern können durch Verdünnen mit Wasser Suspensionen jeder gewünschten Wirkstoffkonzentration erhalten werden. Derartige Suspensionen werden zur Bekämpfung von Unkräutern und Ungräsern in Kulturpflanzungen im Vorauflaufverfahren und zur Behandlung von Rasenanlagen verwendet.

**Paste**

Zur Herstellung einer 45%igen Paste werden folgende Stoffe verwendet:

| | | |
|---|---|---|
| 45 | Teile | Wirkstoff des Beispiels 5 |
| 5 | Teile | Natriumaluminiumsilikat, |
| 14 | Teile | Cetylpolyäthylenglykoläther mit 8 Mol Aethylenoxid, |
| 1 | Teil | Oleylpolyäthylenglykoläther mit 5 Mol Aethylenoxid, |
| 2 | Teile | Spindelöl, |
| 23 | Teile | Wasser, |
| 10 | Teile | Polyäthylenglykol. |

Der Wirkstoff wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen. Die Suspensionen eignen sich zur Behandlung von Rasenanlagen.

Emulsionskonzentrat

Zur Herstellung eines 25%igen Emulsionskonzentrates werden

| | | |
|---|---|---|
| 25 | Teile | eines Wirkstoffs der Formel I |
| 5 | Teile | Mischung von Nonylphenolpolyoxyäthylen und Calcium-dodecylbenzol-sulfonat, |
| 35 | Teile | 3,5,5-Trimethyl-2-cyclohexen-1-on, |
| 35 | Teile | Dimethylformamid |

miteinander vermischt. Dieses Konzentrat kann mit Wasser zu Emulsionen auf geeignete Konzentrationen verdünnt werden.

Anstatt des jeweiligen in den vorhergehenden Formulierungsbeispielen angegebenen Wirkstoffs kann auch eine andere der von der Formel I umfassten Verbindungen verwendet werden.

Wässeriges Konzentrat

Zur Herstellung eines 25%igen wässerigen Konzentrats werden

| | | |
|---|---|---|
| 25 | Teile | Wirkstoff des Beispiels 6 |
| 8 | Teile | Di(-2-Hydroxyäthyl)amin |
| 1 | Teil | Octylphenolpolyglycoläther, |
| 66 | Teile | Wasser |

mit einander vermischt. Dieses Konzentrat kann mit Wasser auf eine geeignete Konzentration verdünnt werden.

Die in den erfindungsgemässen Mitteln enthaltenen Wirkstoffe beeinflussen das Pflanzenwachstum in verschiedener Weise. So hemmen, verzögern oder unterbinden sie in erster Linie das Wachstum und die Keimung. Es handelt sich dabei also sowohl um pre- und post-emergente Herbizidwirkung als auch um Wuchshemmung.

Erfindungsgemässe Mittel, die als aktive Komponente mindestens eine Verbindung der Formel I enthalten, eignen sich besonders zur Hemmung und Kontrolle des Pflanzenwachstums von monocotylen und dicotylen Pflanzen, wie Gräsern, Sträuchern, Bäumen, Getreide- und Leguminosenkulturen, Zuckerrohr, Tabak, Soja, Zwiebel- und Kartoffelknollen, Zierpflanzen, Obstbäumen und Reben.

Die von den neuen Wirkstoffen der Formel I in erster Linie erzielte Wirkung besteht in der gewünschten Reduktion der Pflanzengrösse, insbesondere der Wuchshöhe. Im allgemeinen ist damit eine gewisse Aenderung der Pflanzenform verbunden. In unmittelbarem Zusammenhang zur Verminderung der Wuchshöhe erfährt die Pflanze eine Festigung.Blätter und Stengel sind kräftiger ausgebildet. Durch Verkürzung der Internodienabstände an monocotylen Pflanzen wird die Knickfestigkeit erhöht. Auf diese Weise können Ernteausfälle durch Gewittersturm, Dauerregen, usw., die normalerweise zu einem Lagern von Getreide- und Leguminosenkulturen führen, weitgehend verhindert und damit die Erntearbeit erleichtert werden. Als Nebeneffekt führt verminderte Wuchshöhe bei Nutzpflanzen zu einer Einsparung an Düngemitteln. In gleichem Masse gilt dies auch für Zierpflanzen, Zierrasen, Sportrasen oder sonstige Grünanpflanzungen.

Eines der wichtigsten Probleme an reinen Grasbepflanzungen ist jedoch der Grasschnitt selbst, sei es an Grünanlagen in Wohngegenden, auf Industriegeländen, auf Sportplätzen, an Autostrassen, Flugpisten, Eisenbahndämmen oder Uferböschungen von Gewässern. In all diesen Fällen ist ein periodisches Schneiden des Rasens bzw. des Graswuchses notwendig. Dies ist nicht nur im Hinblick auf Arbeitskräfte und Maschinen sehr aufwendig, sondern bringt im Verkehrsbereich auch erhebliche Gefahren für das betroffene Personal und die Verkehrsteilnehmer mit sich.

Es besteht daher gerade in Gebieten mit grossen Verkehrsnetzen ein dringendes Bedürfnis, die im Hinblick auf die Verfestigung von Seitenstreifen und Böschungen an Verkehrswegen notwendige Grasnarbe einerseits zu erhalten und zu pflegen, andererseits aber mit einfachen Massnahmen während der gesamten Vegetationsperiode auf einer mittleren Wuchshöhe zu halten. Dies wird durch Applikation erfindungsgemässer Wirkstoffe der Formel I auf sehr günstige Weise erreicht.

In analoger Weise kann durch Behandlung von Bäumen, Sträuchern und Hecken, vor allem in Wohn- und Industriegebieten, mit erfindungsgemässen Verbindungen der Formel I die arbeitsaufwendige Schnittarbeit reduziert werden.

Durch den Einsatz erfindungsgemässer Wirkstoffe der Formel I können auch das Triebwachstum und/oder die Fruchtbarkeit von Obstbäumen und Reben vorteilhaft beeinflusst werden.

Zierpflanzen mit starkem Längenwachstum können durch Behandlung mit erfindungsgemässen Wirkstoffen als kompakte Topfpflanzen gezogen werden.

Die Wirkstoffe der Formel I finden auch Anwendung zur Hemmung des Wachstums unerwünschter Geiztriebe, z.B. bei Tabak und Zierpflanzen, wodurch das arbeitsintensive Ausbrechen dieser Triebe von Hand vermieden wird, ferner zur Austriebhemmung bei lagernden Knollen, beispielsweise bei Zierpflanzenknollen, bei Zwiebeln und Kartoffeln, und schliesslich zur Ertragssteigerung bei stark vegetativ wachsenden Kulturpflanzen, wie Soja und Zuckerrohr, indem durch Applikation erfindungsgemässer Wirkstoffe der Uebergang von der vegetativen zur generativen Wachstumsphase beschleunigt wird.

Bevorzugt setzt man die erfindungsgemässen Wirkstoffe der Formel I zur Wachstumshemmung an Gräsern, Getreidekulturen, Tabak, Soja und Zierpflanzen ein.

Die Aufwandmengen sind verschieden und vom Applikationszeitpunkt abhängig. Sie liegen im allgemeinen zwischen 0,1 und 5 kg Wirkstoff pro Hektar, bei Applikation vor dem Auflaufen der Pflanzen und für die Behandlung von bestehenden Kulturen vorzugsweise bis zu 4 kg pro Hektar.

Die Entfaltung der Wirkung der erfindungsgemässen Wirkstoffe erfolgt sowohl über die oberirdischen Pflanzenteile (Kontaktwirkung), insbesondere die Blätter, als auch über den Boden, als pre-emergentes Herbizid (Keimhemmung).

Die Wirkung als starke Wachstumshemmer zeigt sich darin, dass die meisten post-emergent behandelten Pflanzenarten nach dreiwöchiger Versuchsdauer einen Wachstumsstillstand zeigen, wobei die behandelten Pflanzenteile eine dunkelgrüne Färbung annehmen. Die Blätter fallen aber nicht ab.

Diese Wuchshemmung tritt bei einigen Pflanzenarten schon bei einer Dosierung von 0,5 kg/ha und darunter auf.

Da nicht alle Pflanzenarten gleich stark gehemmt werden, ist bei Wahl einer bestimmten niederen Dosierung ein selektiver Einsatz möglich.

Viele der erfindungsgemässen Wirkstoffe sind in pre-emergenter Anwendung gegen viele Unkräuter schon bei relativ niederen Konzentrationen auch hochaktive Herbizide, sowohl gegen Monocotyledonen wie Dicotyledonen. Bei mittleren Konzentrationen sind sie auch herbizid stark wirksam in post-emergenter Anwendung. Viele Wirkstoffe neigen zu totalherbizider Wirkung, andere eignen sich als gute Selektivherbizide, insbesondere in Zuckerrübenkulturen.

In Gebieten mit erhöhter Erosionsgefahr können die erfindungsgemässen Wirkstoffe als Wuchshemmer in den verschiedensten Kulturen eingesetzt werden.

Dabei wird die Unkrautdecke nicht beseitigt, sondern nur so stark gehemmt, dass keine Konkurrenzierung der Kulturpflanzen mehr auftritt.

Die neuen Wirkstoffe der Formel I zeichnen sich überdies durch eine sehr starke pre-emergente Herbizidwirkung aus, sind also auch ausgeprägte Keimungshemmer.

Zum Nachweis der Brauchbarkeit als Herbizide (pre- und post-emergent) und als Wuchshemmer dienten folgende Testmethoden:

<u>Pre-emergente Herbizid-Wirkung</u> (Keimungshemmung)

Im Gewächshaus wird unmittelbar nach der Einsaat der Versuchspflanzen in Saatschalen die Erdoberfläche mit einer wässerigen Suspension der Wirkstoffe, erhalten aus einem 25%-igen Spritzpulver, behandelt. Es wurden verschiedene Konzentrationsreihen angewendet, von 0,05 bis 4 kg Wirksubstanz pro Hektar. Die Saatschalen werden im Gewächshaus bei 22-25°C und 50-70% rel. Luftfeuchtigkeit gehalten und der Versuch nach 3 Wochen ausgewertet und die Resultate nach folgender Notenskala bonitiert:

1 = Pflanzen nicht gekeimt oder total abgestorben
2-8 = Zwischenstufen der Schädigung
9 = Pflanzen ungeschädigt (wie unbehandelte Kontrolle).

als Versuchspflanzen dienen:

| | |
|---|---|
| hordeum (Gerste | setaria italica |
| triticum (Weizen) | echinochloa crus galli |
| zea (Mais) | beta vulgaris |
| sorghum hybr. (Hirse) | sida spinosa |
| oryza (Reis) | sesbania exaltata |

glycine (Soja)
gossypium (Baumwolle)
avena fatua
lolium perenne
alopecurus myosuroides
bromus tectorum
cyperus esculentus
rottboellia exaltata
digitaria sanguinalis
amaranthus retroflexus
sinapis alba
ipomoea purpurea
galium aparine
pastinaca sativa
rumex sp.
chrysanthemum leucum.
abutilon sp.
solanum nigrum

Viele Wirkstoffe ergaben sogar bei einer Dosierung von 0,1 kg/ha eine praktisch vollständige Keimhemmung bei sehr vielen Versuchspflanzen und sind den Wirkstoffen des USP 3 920 444 und der DOS 2 364 144 klar überlegen.

Post-emergente Herbizid-Wirkung (Kontaktherbizid)

Eine grössere Anzahl (mindestens 7) Unkräuter und Kulturpflanzen, sowohl monocotyle wie dicotyle, wurden nach dem Auflaufen (im 4-bis-6-Blattstadium) mit einer wässerigen Wirkstoffemulsion in Dosierungen von 0,05 bis 4 kg Wirksubstanz pro Hektar auf die Pflanzen gespritzt und diese bei 24°-26°C und 45-60% rel. Luftfeuchtigkeit gehalten. 5 Tage und 15 Tage nach Behandlung wird der Versuch ausgewertet und das Ergebnis wie im pre-emergent-Versuch nach derselben Notenskala bonitiert.

Die geprüften Verbindungen gemäss vorliegender Erfindung zeigten auf einigen Pflanzen ausgeprägte kontaktherbizide Wirkung und auf vielen Pflanzen Wachstumsstillstand als Symptom der wachstumshemmenden Eigenschaften.

Wuchshemmung bei Gräsern

In Kunststoffschalen mit Erde-Torf-Sand-Gemisch (6:3:1) wurden Samen der Gräser Lolium perenne, Poa pratensis, Festuca ovina und Dactylis glomerata ausgesät und normal bewässert. Die aufgelaufenen Gräser wurden wöchentlich bis auf 4 cm Höhe zurückgeschnitten und 40 Tage nach der Aussaat und 1 Tag nach dem letzten Schnitt mit wässerigen Spritzbrühen eines Wirkstoffs der Formel I bespritzt. Die Wirkstoffmenge betrug umgerechnet 0,5 bis 5 kg Aktivsubstanz pro Hektar. 10 und 21 Tage nach Applikation wurde das Wachstum der Gräser beurteilt.

Wuchshemmung bei Getreide

In Kunststoffbechern wurde Sommerweizen (Triticum aestivum), Sommergerste (Hordeum vulgare) und Roggen (Secale) in sterilisierter Erde angesät und im Gewächshaus gezogen. Die Getreidesprösslinge werden 5 Tage nach Aussaat mit einer Spritzbrühe des Wirkstoffs behandelt. Die Blattapplikation entsprach 0,1 bis 5 kg Wirkstoff pro Hektar. Die Auswertung erfolgt nach 21 Tage.

Die erfindungsgemässen Wirkstoffe bewirken eine merkliche Wuchshemmung sowohl bei den Gräsern wie beim Getreide.

Patentansprüche

1. Neue N-phenylsubstituierte N-Heterocyclen der Formel I

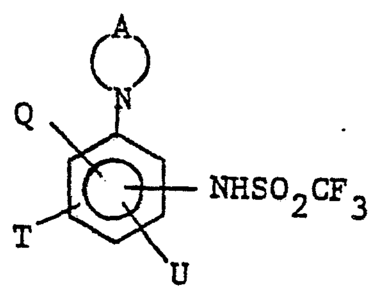

(I)

und ihre Salze, worin

A drei bis sechs Glieder eines gesättigten oder ein- bis dreifach ungesättigten heterocyclischen Ringes darstellt, und mindestens eines und höchstens drei dieser Glieder gegebenenfalls substituierte Heteroatome aus der Gruppe O, S, N und P sind und die restlichen Glieder gegebenenfalls substituierte Kohlenstoff-Kettenglieder darstellen, wobei noch ein weiterer Ring ankondensiert vorhanden sein kann, mit der Massgabe, dass bei Vorhandensein von O bezw. S als Heteroatome höchstens zwei derselben benachbart stehen,

Q, T und U unabhängig voneinander je Wasserstoff, Halogen, $C_1$-$C_4$ Alkyl oder Halogenalkyl, Cyano, Nitro, $-CSNH_2$, gegebenenfalls substituiertes Benzyl oder eine Gruppe $-S(O)_n-R_a$, $-OR_b$, $-COOR_c$, $-SO_2-N(R_d)_2$ oder $-N(R_e)_2$ bedeuten, wobei

n eine Zahl von Null bis 2 ist,

$R_a$ einen $C_1$-$C_5$ Alkylrest oder einen gegebenenfalls kernsubstituierten Phenyl- oder Phenylalkylrest,

$R_b$ $C_1$-$C_5$ Alkyl, Alkoxyalkyl, Alkoxyalkoxyalkyl, Alk(en)ylcarbonyl oder gegebenenfalls durch niederes Alkyl, Halogen, CN, $CF_3$ oder $NO_2$ substituiertes Phenyl oder Benzyl,

$R_c$ Wasserstoff oder nieder Alkyl,

$R_d$ nieder Alkyl, und

$R_e$ Wasserstoff oder nieder Alkyl darstellen.

2. Neue substituierte N-Heterocyclen der Formel I des Patentanspruchs 1, dadurch gekennzeichnet, dass sie den engeren Formeln IIa oder IIb entsprechen:

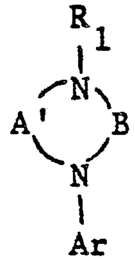
(IIa)

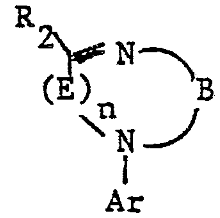
(IIb)

worin die neuen und schon in Formel I verwendeten Symbole folgende Bedeutungen haben:

Ar steht für den substituierten Phenylrest der Formel I

A' ist eine Gruppe $\geq C=O$, eine gegebenenfalls durch $R_2$ oder $R_3$ substituierte $C_1-C_3$ Alkylen- oder Alkenylen-Gruppe oder ein Rest -CO-D, worin D gegebenenfalls durch $R_2$ und/oder $R_3$ substituiertes $C_1-C_2$ Alkylen oder $C_2$-Alkenylen darstellt, $R_2$ und $R_3$ neben Wasserstoff einen gegebenenfalls durch $C_1-C_4$ Alkoxy oder durch CN substituierten $C_1-C_8$ Alkyl, $C_1-C_4$ Alkoxy-, einen Cyano- oder $-S(O)_n$-Alkylrest bedeuten, n eine Zahl von Null bis 2 ist, $R_2$ und $R_3$ zusammen auch einen $C_2-C_6$ Alkylen, $C_2-C_6$ Alkenylen oder $C_4$-Alkadienylenrest zwecks Bildung eines weiteren Ringes bedeuten können, der selbst wiederum durch $R_2$ bezw. $R_3$ substituiert sein kann,

B hat entweder die gleiche Bedeutung wie A' oder ist eine Gruppe -CO-CO- oder $\geq C=X$, worin X Schwefel oder $=NR_4$ bedeutet;

E ist gegebenenfalls durch $R_2$ und/oder $R_3$ substituiertes $C_2$-Alkenylen, die Gruppe $\geq C=O$ oder $\geq C<^{R_2}_{R_3}$ und n ist eine Zahl von Null bis 2,

$R_1$ ist $C_1-C_8$ Alkyl (auch $R_5$ genannt), Wasserstoff, $C_3-C_8$ Cycloalkyl, wobei Alkyl- und Cycloalkylreste gegebenenfalls substituiert sein können durch Halogen, $C_1-C_4$ Alkoxy, durch $-N<^{R_7}_{R_6}$, CN, $-COOR_8$,

oder gegebenenfalls substituiertes Phenyl, $C_3$-$C_8$ Alkenyl oder Halogenalkenyl, $C_3$-$C_8$ Alkinyl, gegebenenfalls durch Alkyl, Alkoxy, Halogen, $CF_3$ oder CN substituiertes Phenyl, ferner eine der Gruppen $-CON<^{R_6}_{R_7}$, $-COOR_8$ oder $COR_1$, wobei $R_1$ wieder die obige Bedeutung hat,

$R_4$ ist $C_1$-$C_8$ Alkyl, Benzyl oder Phenyl,

$R_6$ und $R_7$ bedeuten unabhängig voneinander je Wasserstoff, gegebenenfalls durch $C_1$-$C_4$ Alkoxy substituiertes $C_1$-$C_8$ Alkyl, $C_3$-$C_8$ Alkenyl oder Alkinyl, $C_1$-$C_4$ Alkoxy, Phenyl oder Benzyl, wobei der Benzolkern dieser Reste durch Halogen, $C_1$-$C_4$ Alkyl, $C_1$-$C_4$ Alkoxy oder durch CN substituiert sein kann,

$R_6$ und $R_7$ können zusammen mit dem benachbarten Stickstoffatom auch einen 5-6-gliedrigen heterocyclischen Ring bilden, der noch ein weiteres Sauerstoffatom als Heteroatom enthalten kann,

$R_8$ ist Wasserstoff, $C_1$-$C_8$ Alkyl, $C_3$-$C_8$ Cycloalkyl, gegebenenfalls durch $C_1$-$C_4$ Alkoxy oder Halogen substituiertes Aralkyl (insbesondere Benzyl oder Phenyläthyl), ferner gegebenenfalls durch Halogen, Alkyl, Alkoxy, $CF_3$ oder CN substituiertes Phenyl.

3. Neue substituierte N-Heterocyclen gemäss Patentansprüchen 1 und 2, in denen die Gruppe $-NHSO_2CF_3$ sich in der meta-Stellung des Phenylrestes befindet.

4. Verfahren zur Herstellung der neuen phenylsubstituierten N-Heterocyclen der Formel I des Patentanspruch 1, dadurch gekennzeichnet, dass man ein heterocyclisches Aminoanilinderivat der Formel III

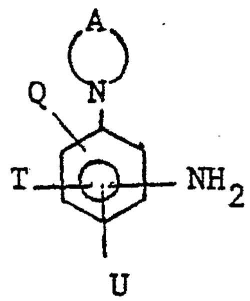

(III)

worin A, Q, T und U wie unter Formel I definiert sind, in an sich bekannter Weise mit einem Trifluormethansulfonylierungsmittel behandelt und das so erhaltene Trifluormethansulfonamid gewünschtenfalls in das Salz einer Base oder eines Amins überführt.

5. Verfahren gemäss Patentanspruch 4, dadurch gekennzeichnet, dass man als Trifluormethansulfonylierungsmittel das Anhydrid der Trifluormethansulfonsäure von der Formel $(CF_3SO_2)_2O$ verwendet.

6. Mittel zur Beeinflussung des Pflanzenwachstums, insbesondere herbizides und pflanzenwuchshemmendes Mittel, dadurch gekennzeichnet, dass es neben Träger- und/oder anderen Zuschlagstoffen als aktive Komponente mindestens eine trifluormethansulfonamidophenyl-substituierte heterocyclische Verbindung der Formel I, gemäss der Definition des Anspruchs 1 oder 2 enthält.

7. Verfahren zur Hemmung und Unterdrückung des Pflanzenwuchses von monocotylen und dicotylen Pflanzen, gekennzeichnet durch pre- oder post-emergente Behandlung der besäten Flächen oder der Pflanzen mit einem Wirkstoff der Formel I, II oder IIa gemäss Ansprüchen 1 und 2, oder mit einem diesen Wirkstoff enthaltenden Mittel gemäss Anspruch 6.

8. Verfahren zur Wuchshemmung an Gräsern, Getreidekulturen, Tabak, Soja und Zierpflanzen, gekennzeichnet durch die post-emergente Behandlung dieser Pflanzen mit einem Wirkstoff der Formel I, IIa oder IIb gemäss Ansprüchen 1 und 2 oder mit einem Mittel gemäss Anspruch 6.

0011693

Nummer der Anmeldung

EP 79103734.4

Europäisches Patentamt

# EUROPÄISCHER RECHERCHENBERICHT

| EINSCHLÄGIGE DOKUMENTE | | | KLASSIFIKATION DER ANMELDUNG (Int.Cl.) 3 |
|---|---|---|---|
| Kategorie | Kennzeichnung des Dokuments mit Angabe, soweit erforderlich, der maßgeblichen Teile | betrifft Anspruch | |
| | DE - A1 - 2 650 604 (CIBA-GEIGY)<br>+ Patentansprüche; Seiten 22, 23 +<br>-- | 1-3, 6-8 | C 07 D 233/36<br>C 07 D 233/70<br>C 07 D 233/72<br>C 07 D 239/22<br>C 07 D 249/12<br>C 07 D 263/20<br>C 07 D 263/44<br>C 07 D 275/04<br>A 01 N 43/50<br>A 01 N 43/54<br>A 01 N 43/64<br>A 01 N 43/76<br>A 01 N 43/80 |
| | DE - A1 - 2 542 453 (BASF)<br>+ Patentansprüche; Seiten 26 bis 28 +<br>-- | 1-3 | |
| | DE - A - 2 212 558 (CIBA-GEIGY)<br>+ Patentanspruch 1, Seiten 12 - 17 +<br>-- | 1-3, 6-8 | RECHERCHIERTE SACHGEBIETE (Int. Cl.) 3 |
| | US - A - 4 076 519 (HARRINGTON)<br>+ Abstract, Spalten 5,6,9-17 +<br>-- | 1-8 | C 07 D 231/00<br>C 07 D 233/00<br>C 07 D 235/00<br>C 07 D 237/00<br>C 07 D 239/00<br>C 07 D 241/00<br>C 07 D 243/00<br>C 07 D 249/00<br>C 07 D 263/00<br>C 07 D 271/00<br>C 07 D 275/00<br>C 07 D 279/00<br>A 01 N 43/00 |
| | US - A - 3 923 811 (HARRINGTON)<br>+ Spalten 1-5 +<br>-- | 1-8 | |
| | CHEMICAL ABSTRACTS, Vol. 81 (1974)<br>+ 105011 e +<br>-- | 1-3, 6-8 | |
| | CHEMICAL ABSTRACTS, Vol. 84 (1976)<br>+ 43626 k +<br>---- | 1-3, 6-8 | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |
| X | Der vorliegende Recherchenbericht wurde für alle Patentansprüche erstellt. | | |

| Recherchenort | Abschlußdatum der Recherche | Prüfer |
|---|---|---|
| WIEN | 19-12-1979 | HOCHHAUSER |